# FASCICULE DE BREVET EUROPEEN

(11) **EP 1 733 714 B1**
(45) Date de publication et mention de la délivrance du brevet: **19.05.2010**
(21) Numéro de dépôt: 06114654.4
(22) Date de dépôt: 29.05.2006
(51) Int. Cl.: A61K 8/49, A61Q 5/10, A61K 8/34, A61K 8/39

(54) **Composition pour la teinture des fibres kératiniques comprenant un dérivé de diamino-N,N-dihydro-pyrazolone, un coupleur et un polyol**
Mittel zum Färben von Keratinfasern enthaltend ein Diamino-N,N-Dihydropyrazolonderivat, einen Kuppler und ein Polyol
Composition for dyeing keratinic fibres comprising a diamino-N,N-dihydro-pyrazolone derivative, a coupling agent and a polyol

(30) Priorité: 31.05.2005 FR 0551445
(43) Date de publication de la demande: 20.12.2006
(73) Titulaire: L'Oréal, 75008 Paris (FR)
(72) Inventeur: Saunier, Jean-Baptiste, 75014 Paris (FR)
(74) Mandataire: Prevel, Estelle Nicole

(56) Documents cités:
- EP-A- 1 464 327
- EP-A- 1 550 656
- DE-A1- 1 617 893
- DE-A1- 10 148 847

## Description

L'invention a pour objet une composition pour la teinture des fibres kératiniques, et en particulier des fibres kératiniques humaines telles que les cheveux, comprenant au moins une base d'oxydation du type diamino-N,N-dihydropyrazolone ou un de ses sels d'addition, au moins un coupleur et au moins un polyol en C₄-C₃₀, ainsi que le procédé de coloration mettant en oeuvre une telle composition.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés d'indole, des dérivés d'indoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants. On obtient ainsi des colorations permanentes.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques.

La variété des molécules mises en jeu au niveau des bases d'oxydation et des coupleurs permet l'obtention d'une riche palette de couleurs.

L'utilisation de bases d'oxydation telle que les dérivés de para-phénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, de variété de nuances, d'uniformité de la couleur et de la ténacité aux agents extérieurs.

L'utilisation de ces bases à pH neutre ne permet pas d'atteindre une gamme de nuances variées, en particulier pour les nuances chaudes telles que les rouges et les orangés.

Le but de la présente invention est de fournir de nouvelles compositions de coloration des fibres kératiniques qui permettent d'obtenir une coloration aux nuances variées, puissante, chromatique, esthétique, peu sélective et résistant bien aux diverses agressions que peuvent subir les cheveux tels que les shampooings, la lumière, la sueur et les déformations permanentes.

La présente invention a donc pour objet une composition de coloration des fibres kératiniques comprenant, dans un milieu approprié :
- au moins une base d'oxydation choisie parmi un dérivé de la diamino-N,N-dihydro-pyrazolone de formule (I) ou l'un de ses sels d'addition : 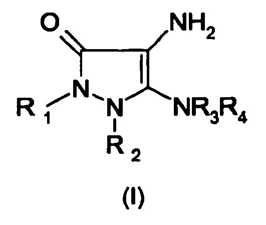
dans laquelle :
R₁, R₂, R₃ et R₄, identiques ou différents, représentent :
   - un radical alkyle en C₁-C₁₀, de préférence en C₁-C₆, linéaire ou ramifié éventuellement substitué par un ou plusieurs radicaux choisis dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un ou plusieurs groupes (C₁-C₄)alkyle, hydroxy, alcoxy en C₁-C₂, amino, (di)alkyl(C₁-C₂)amino ;
   - un radical aryle éventuellement substitué par un ou plusieurs radicaux (C₁-C₄)alkyle, hydroxy, alcoxy en C₁-C₂, amino, (di)alkyl(C₁-C₂)amino ;
   - un radical hétéroaryle à 5 ou 6 chaînons, éventuellement substitué par un ou plusieurs radicaux choisis parmi un radical (C₁-C₄)alkyle, (C₁-C₂)alcoxy ;
R₃ et R₄ peuvent représenter également un atome d'hydrogène ;
R₅, R₆ et R₇, identiques ou différents, représentent :
   - un atome d'hydrogène ;
   - un radical alkyle linéaire ou ramifié en C₁-C₄ éventuellement substitué par un ou plusieurs radicaux choisis parmi un radical hydroxy, alcoxy en C₁-C₂, carboxamido CONR₈R₉, sulfonyle SO₂R₈, aryle éventuellement substitué par un radical (C₁-C₄)alkyle, hydroxy, alcoxy en C₁-C₂, amino, (di)alkyl(C₁-C₂)amino; aryle éventuellement substitué par un radical (C₁-C₄)alkyle, hydroxy, alcoxy en C₁-C₂, amino, (di)alkyl(C₁-C₂)amino ;
R₆ et R₇, identiques ou différents, peuvent représenter également un radical carboxamido CONR₈R₉; un radical sulfonyle SO₂R₈;
R₈ et Rg, identiques ou différents, représentent un atome d'hydrogène; un radical alkyle en C₁-C₄ linéaire ou ramifié éventuellement substitué par un ou plusieurs radicaux hydroxy, alcoxy en C₁-C₂ ;
R₁ et R₂ d'une part, et R₃ et R₄ d'autre part, peuvent former avec le ou les atomes d'azote auxquels ils sont rattachés un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyle en C₁-C₄ éventuellement substitués par un ou plusieurs radicaux hydroxy, amino, (di)-alkylamino, alcoxy, carboxy, sulfonyle ;
R₃ et R₄ peuvent également former ensemble avec l'atome d'azote auquel ils sont rattachés un hétérocycle à 5 ou 7 chaînons dont les atomes de carbone peuvent être remplacés par un atome d'oxygène ou d'azote éventuellement substitué ;
   - au moins un coupleur ; et
   - au moins un polyol en C₄-C₃₀.

La présente invention permet en particulier d'obtenir une coloration des fibres kératiniques aux nuances variées, puissante, esthétique, peu sélective et résistant bien aux diverses agressions que peuvent subir les cheveux tels que les shampooings, la lumière, la sueur et les déformations permanentes. Elle permet de plus d'obtenir des colorations intenses et variées à pH neutre.

Un autre objet de l'invention est un procédé de teinture des fibres kératiniques mettant en oeuvre la composition de la présente invention, ainsi que l'utilisation de cette composition pour la teinture des fibres kératiniques.

L'invention a enfin pour objet un kit de coloration comprenant d'une part une composition de coloration contenant une base d'oxydation de formule (I), un coupleur et un polyol en C₄-C₃₀ et d'autre part une composition contenant un agent oxydant.

Dans le cadre de l'invention, on entend par radical alkyle des radicaux alkyles linéaires ou ramifiés en C₁-C₁₀ sauf indication contraire, préférentiellement en C₁-C₆, encore plus préférentiellement en C₁-C₄, tels que le radical méthyle, éthyle, propyle, isopropyle, isobutyle, tertiobutyle, pentyle, hexyle.

Plus particulièrement, dans la formule (I), les radicaux R₁ et R₂, identiques ou différents, sont choisis parmi :
- un radical alkyle en C₁-C₆ éventuellement substitué par un radical hydroxy, (C₁-C₂)alcoxy, amino, (di)alkyl(C₁-C₂)amino ;
- un radical phényle, méthoxyphényle, éthoxyphényle, benzyle.

De préférence, les radicaux R₁ et R₂, identiques ou non, sont choisis parmi un radical méthyle, éthyle, 2-hydroxyéthyle, 3-hydroxypropyle, 2-hydroxypropyle, phényle.

Selon un autre mode de réalisation, les radicaux R₁ et R₂ forment ensemble avec les atomes d'azote auxquels ils sont rattachés un cycle à 5 ou 6 chaînons, saturé ou insaturé, éventuellement substitué.

De préférence, les radicaux R₁ et R₂ forment ensemble avec les atomes d'azote auxquels ils sont rattachés un cycle pyrazolidine, pyridazolidine, éventuellement substitué par un ou plusieurs radicaux alkyle en C₁-C₄, hydroxy, (C₁-C₂)alcoxy, carboxy, carboxamido, amino, (di)alkyl(C₁-C₂)amino.

De manière encore plus avantageuse, les radicaux R₁ et R₂ forment ensemble avec les atomes d'azote auxquels ils sont rattachés un cycle pyrazolidine, pyridazolidine.

En ce qui concerne les radicaux R₃ et R₄, ces derniers, identiques ou différents, sont plus particulièrement choisis parmi un atome d'hydrogène ; un radical alkyle en C₁-C₆ linéaire ou ramifié, éventuellement substitué par un ou plusieurs radicaux hydroxy, (C₁-C₂)alcoxy, amino, (di)alkyl(C₁-C₂)amino ; un radical phényle éventuellement substitué par un ou plusieurs radicaux hydroxy, amino, (C₁-C₂)alcoxy.

De préférence, les radicaux R₃ et R₄, identiques ou non, sont choisis parmi un atome d'hydrogène, un radical méthyle, éthyle, isopropyle, 2-hydroxyéthyle, 3-hydroxypropyle, 2-hydroxypropyle, 2-carboxyéthyle. Selon un mode de réalisation particulier, les radicaux R₃ et R₄ représentent un atome d'hydrogène.

Selon un autre mode de réalisation, les radicaux R₃ et R₄ forment ensemble avec l'atome d'azote auquel ils sont rattachés un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl(C₁-C₂)amino, carboxy, carboxamido, alkyle en C₁-C₄ éventuellement substitué par un ou plusieurs radicaux hydroxy, amino, (di)alkylamino en C₁-C₂.

Plus particulièrement, les radicaux R₃ et R₄ forment ensemble avec l'atome d'azote auquel ils sont rattachés un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino- pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

De préférence, les radicaux R₃ et R₄ forment ensemble avec l'atome d'azote auquel ils sont rattachés un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthyl homopipérazine, la N β-hydroxyéthylhomopipérazine.

Conformément à un mode de réalisation encore plus préféré de l'invention, les radicaux R₃ et R₄ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

Les composés de formule (I) peuvent être éventuellement salifiés par des acides minéraux forts tels que par exemple HCl, HBr, HI, H₂SO₄, H₃PO₄, ou des acides organiques tels que, par exemple, l'acide acétique, lactique, tartrique, citrique ou succinique, benzènesulfonique, para-toluènesulfonique, formique, méthanesulfonique.

Ils peuvent aussi être sous forme de solvates par exemple un hydrate ou un solvate d'alcool linéaire ou ramifié tel que l'éthanol ou l'isopropanol.

A titre d'exemples de dérivés de formule (I), on peut citer les composés présentés ci-dessous ou leurs sels d'addition :
4,5-diamino-1,2-diméthyl-1,2-dihydro-pyrazol-3-one ;
4-amino-5-méthylamino-1,2-diméthyl-1,2-dihydro-pyrazol-3-one ;
4-amino-5-diméthylamino-1,2-diméthyl-1,2-dihydro-pyrazol-3-one ;
4-amino-5-(2-hydroxyéthyl)amino-1,2-diméthyl-1,2-dihydro-pyrazol-3-one ;
4-amino-5-(pyrrolidin-1-yl)-1,2-diméthyl-1,2-dihydro-pyrazol-3-one ;
4-amino-5-(pipéridin-1-yl)-1,2-diméthyl-1,2-dihydro-pyrazol-3-one ;

4,5-diamino-1,2-di-(2-hydroxyéthyl)-1,2-dihydro-pyrazol-3-one ;
4-amino-5-méthylamino-1,2-di-(2-hydroxyéthyl)-1,2-dihydro-pyrazol-3-one ;
4-amino-5-diméthylamino-1,2-di-(2-hydroxyéthyl)-1,2-dihydro-pyrazol-3-one ;
4-amino-5-(2-hydroxyéthyl)amino-1,2-di-(2-hydroxyéthyl)-1,2-dihydro-pyrazol-3-one ;
4-amino-5-(pyrrolidin-1-yl)-1,2-di-(2-hydroxyéthyl)-1,2-dihydro-pyrazol-3-one ;
4-amino-5-(pipéridin-1-yl)-1,2-di-(2-hydroxyéthyl)-1,2-dihydro-pyrazol-3-one ;

4,5-diamino-1,2-diéthyl-1,2-dihydro-pyrazol-3-one ;
4,5-diamino-1,2-phényl-1,2-dihydro-pyrazol-3-one ;
4,5-diamino-1-éthyl-2-méthyl-1,2-dihydro-pyrazol-3-one ;
4,5-diamino-2-éthyl-1-méthyl-1,2-dihydro-pyrazol-3-one ;
4,5-diamino-1-phényl-2-méthyl-1,2-dihydro-pyrazol-3-one ;
4,5-diamino-2-phényl-1-méthyl-1,2-dihydro-pyrazol-3-one ;
4,5-diamino-1-(2-hydroxyéthyl)-2-méthyl-1,2-dihydro-pyrazol-3-one ;
4,5-diamino-2-(2-hydroxyéthyl)-1-méthyl-1,2-dihydro-pyrazol-3-one ;

2,3-diamino-6,7-dihydro-1H,5H-pyrazolo[1,2-a]pyrazol-1-one ;
2-amino-3-méthylamino-6,7-dihydro-1H,5H-pyrazolo[1,2-a]pyrazol-1-one ;
2-amino-3-diméthylamino-6,7-dihydro-1H,5H-pyrazolo[1,2-a]pyrazol-1-one ;
2-amino-3-éthylamino-6,7-dihydro-1H,5H-pyrazolo[1,2-a]pyrazol-1-one ;
2-amino-3-isopropylamino-6,7-dihydro-1H,5H-pyrazolo[1,2-a]pyrazol-1-one ;
2-amino-3-(2-hydroxyéthyl)amino-6,7-dihydro-1H,5H-pyrazolo[1,2-a]pyrazol-1-one ;
2-amino-3-(2-hydroxypropyl)amino-6,7-dihydro-1H,5H-pyrazolo[1,2-a]pyrazol-1-one ;
2-amino-3-bis(2-hydroxyéthyl)amino-6,7-dihydro-1H,5H-pyrazolo[1,2-a]pyrazol-1-one ;

2-amino-3-(pyrrolidin-1-yl)-6,7-dihydro-1H,5H-pyrazolo[1,2-a]pyrazol-1-one;
2-amino-3-(3-hydroxy-pyrrolidin-1-yl)-6,7-dihydro-1H,5H-pyrazolo[1,2-a]pyrazol-1-one;
2-amino-3-(pipéridin-1-yl)-6,7-dihydro-1H,5H-pyrazolo[1,2-a]pyrazol-1-one;

2,3-diamino-6-hydroxy-6,7-dihydro-1H,5H-pyrazolo[1,2-a]pyrazol-1-one;
2,3-diamino-6-méthyl-6,7-dihydro-1H,5H-pyrazolo[1,2-a]pyrazol-1-one;
2,3-diamino-6-diméthyl-6,7-dihydro-1H,5H-pyrazolo[1,2-a]pyrazol-1-one;

2,3-diamino-5,6,7,8-tétrahydro-1H,6H-pyridazino[1,2-a]pyrazol-1-one;
2,3-diamino-5,8-dihydro-1H,6H-pyridazino[1,2-a]pyrazol-1-one;

4-Amino-5-dimethylamino-1,2-diethyl-1,2-dihydro-pyrazol-3-one;
4-Amino-1,2-diethyl-5-ethylamino-1,2-dihydro-pyrazol-3-one;
4-Amino-1,2-diethyl-5-isopropylamino-1,2-dihydro-pyrazol-3-one ;
4-Amino-1,2-diethyl-5-(2-hydroxy-ethylamino)-1,2-dihydro-pyrazol-3-one ;
4-Amino-5-(2-dimethylamino-ethylamino)-1,2-diethyl-1,2-dihydro-pyrazol-3-one ;
4-Amino-5-[bis-(2-hydroxy-ethyl)-amino]-1,2-diethyl-1,2-dihydro-pyrazol-3-one ;
4-Amino-1,2-diethyl-5-(3-imidazol-1-yl-propylamino)-1,2-dihydro-pyrazol-3-one ;
4-Amino-5-dimethylamino-1,2-diethyl-1,2-dihydro-pyrazol-3-one ;
4-Amino-1,2-diethyl-5-ethylamino-1,2-dihydro-pyrazol-3-one ;
4-Amino-1,2-diethyl-5-isopropylamino-1,2-dihydro-pyrazol-3-one ;
4-Amino-1,2-diethyl-5-(2-hydroxy-ethylamino)-1,2-dihydro-pyrazol-3-one ;
4-Amino-5-(2-dimethylamino-ethylamino)-1,2-diethyl-1,2-dihydro-pyrazol-3-one ;
4-Amino-5-[bis-(2-hydroxy-ethyl)-amino]-1,2-diethyl-1,2-dihydro-pyrazol-3-one ;
4-Amino-1,2-diethyl-5-(3-imidazol-1-yl-propylamino)-1,2-dihydro-pyrazol-3-one ;
4-Amino-1.2-diethyl-5-(3-hydroxy-pyrrolidin-1-yl)-1,2-dihydro-pyrazol-3-one ;
4-Amino-1.2-diethyl-5-pyrrolidin-1-yl-1,2-dihydro-pyrazol-3-one ;
4-Amino-5-(3-dimethylamino-pyrrolidin-1-yl)-1,2-diethyl-1,2-dihydro-pyrazol-3-one ;
4-Amino-1,2-diethyl-5-(4-methyl-piperazin-1-yl)-pyrazolidin-3-one ;
2,3-Diamino-6-hydroxy-6,7-dihydro-5H-pyrazolo[1,2-a]pyrazol-1-one ;
dont certains sont figurés ci-dessous pour illustrer les noms par des structures chimiques :

| | |
|---|---|
| 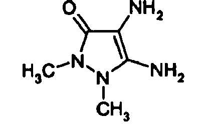 | 4,5-Diamino-1,2-diméthyl-1,2-dihydro-pyrazol-3-one |
| 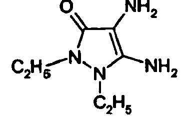 | 4,5-Diamino-1,2-diéthyl-1,2-dihydro -pyrazol-3-one |
| 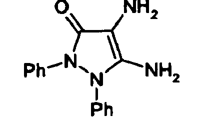 | 4,5-Diamino-1,2-diphényl-1,2-dihydro-pyrazol-3-one |
| 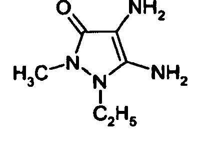 | 4,5-Diamino-1-éthyl-2-méthyl-1,2-di hydro-pyrazol-3-one |
| 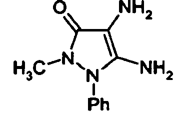 | 4,5-Diamino-1-phényl-2-méthyl-1,2-d ihydro-pyrazol-3-one |
| 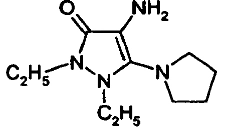 | 4-Ami no-5-(pyrrol idin-1-yl)-1,2-d iethyl-1,2-di hydro-pyrazol-3-one |
| 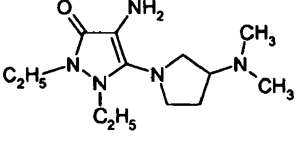 | 4-Amino-5-(3-dimethylamino-pyrrolidin-1-yl)-1,2-diethyl-1,2-dihydro-pyrazol-3-one |
| 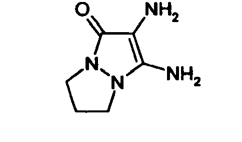 | 2,3-diamino-6,7-dihydro-1H,SH-pyrazolo[1,2-a]pyrazol-1-one |
| 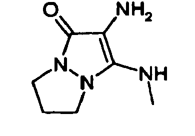 | 2-Amino-3-méthylamino-6,7-dihydro-1H,5H-pyrazolo[1,2-a] pyrazol-1-one |
| 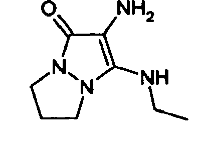 | 2-Amino-3-éthylamino-6,7-dihydro-1H,5H-pyrazolo[1,2-a] pyrazol-1-one |
| 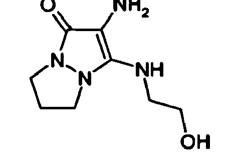 | 2-amino-3-(2-hydroxyéthyl)amino-6,7-dihydro-1H,5H-pyrazolo[1,2-a]pyrazol-1-one |
| 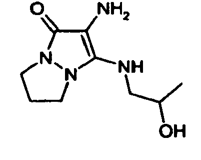 | 2-amino-3-(2-hydroxypropyl)amino-6,7-dihydro-1H,5H-pyrazolo[1,2-a]pyrazol-1-one |
| 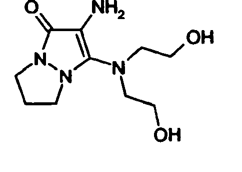 | 2-amino-3-bis(2-hydroxyéthyl)amino-6,7-dihydro-1H,5H-pyrazolo[1,2-a]pyrazol-1-one |
| 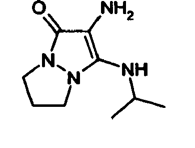 | 2-amino-3-isopropylamino-6,7-dihydro-1H,5H-pyrazolo[1,2-a] pyrazol-1-one |
| 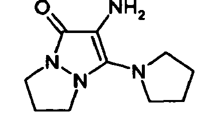 | 2-amino-3-(pyrrolidin-1-yl)-6,7-dihydro-1H,5H-pyrazolo[1,2-a] pyrazol-1-one |
| 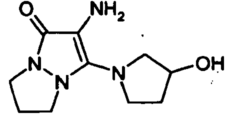 | 2-amino-3-(3-hydroxy-pyrrolidin-1-yl)-6,7-dihydro-1H,5H-pyrazolo[1,2-a]pyrazol-1-one |
| 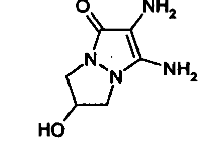 | 2,3-diamino-6-hydroxy-6,7-dihydro-1H,5H-pyrazolo[1,2-a] pyrazol-1-one |
| 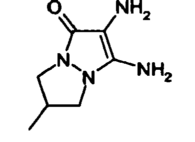 | 2,3-diamino-6-méthyl-6,7-dihydro-1H,5H-pyrazolo[1,2-a] pyrazol-1-one |
| 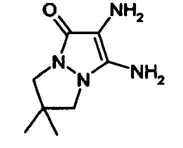 | 2,3-diamino-6,6-diméthyl-6,7-dihydro-1H,5H-pyrazolo[1,2-a]pyrazol-1-one |
| 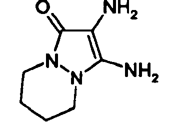 | 2,3-diamino-5,6,7,8-tétrahydro-1H,6H-pyridazino[1,2-a]pyrazol-1-one |
| 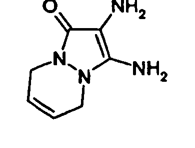 | 2,3-diamino-5,8-dihydro-1H,6H-pyridazino[1,2-a]pyrazol-1-one |
| 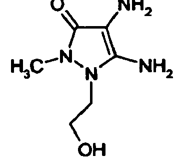 | 4,5-diamino-1-(2-hydroxyéthyl)-2-méthyl-1,2-dihydro-pyrazol-3-one |
| 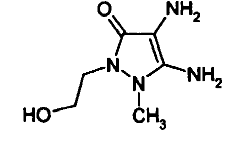 | 4,5-diamino-2-(2-hydroxyéthyl)-1-méthyl-1,2-dihydro-pyrazol-3-one. |

Parmi ces composés, les dérivés de diamino-N,N-dihydropyrazolone de formule (I) ou leurs sels d'addition particulièrement préférés sont les suivants :
2,3-Diamino-6,7-dihydro-1H,5H-pyrazolo[1,2-a]pyrazol-1-one ;
2-Amino-3-éthylamino-6,7-dihydro-1H,5H-pyrazolo[1,2-a]pyrazol-1-one ;
2-Amino-3-isopropylamino-6,7-dihydro-1H,5H-pyrazolo[1,2-a]pyrazol-1-one ;
2-Amino-3-(pyrrolidin-1-yl)-6,7-dihydro-1H,5H-pyrazolo[1,2-a]pyrazol-1-one ;
4,5-diamino-1,2-diméthyl-1,2-dihydro-pyrazol-3-one ;
4,5-diamino-1,2-diéthyl-1,2-dihydro-pyrazol-3-one ;
4,5-diamino-1,2-di-(2-hydroxyéthyl)-1,2-dihydro-pyrazol-3-one ;
2-amino-3-(2-hydroxyéthyl)amino-6,7-dihydro-1H,5H-pyrazolo[1,2-a]pyrazol-1-one ;
2-amino-3-diméthylamino-6,7-dihydro-1H,5H-pyrazolo[1,2-a]pyrazol-1-one ;
2,3-diamino-5,6,7,8-tétrahydro-1H,6H-pyridazino[1,2-a]pyrazol-1-one
4-Amino-1.2-diethyl-5-pyrrolidin-1-yl-1,2-dihydro-pyrazol-3-one ;
4-Amino-5-(3-dimethylamino-pyrrolidin-1-yl)-1,2-diethyl-1,2-dihydro-pyrazol-3-one ;
2,3-diamino-6-hydroxy-6,7-dihydro-1H,5H-pyrazolo[1,2-a]pyrazol-1-one.

La ou les bases d'oxydation de formule (I) sont en général présentes chacune en quantité comprise entre 0,001 et 10 % en poids environ du poids total de la composition tinctoriale, de préférence entre 0,005 et 6 %.

Le ou les coupleurs utiles dans le cadre de l'invention peuvent être choisis parmi les méta-phénylènediamines, les méta-aminophénols, les méta-diphénols, les coupleurs naphtaléniques, les coupleurs hétérocycliques et leurs sels d'addition.

A titre d'exemples, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont en général chacun présents en quantité comprise entre 0,001 et 10 % en poids environ du poids total de la composition tinctoriale, de préférence entre 0,005 et 6 %.

Dans le cadre de la présente invention, on entend par polyol un composé comportant une chaîne hydrocarbonée, linéaire, ramifiée ou cyclique, saturée ou insaturée, portant au moins deux fonctions hydroxyle. Ladite chaîne et ses ramifications peuvent être interrompues par 1 à 6 atomes d'oxygène. Ladite chaîne et ses ramifications peuvent également porter un ou plusieurs substituants autres que les groupements hydroxyle, substituants choisis parmi les groupements carboxy, amino, halogène, aryle en C₆-C₃₀.

A titre d'exemples de polyol en C₄-C₃₀, on peut citer les diols, ramifiés ou non ramifiés, tels que le 1,4-butanediol; le 1,5-pentanediol; le 1,6-hexanediol; le néopentylglycol (ou le 2,3-diméthyl-1,3-propanediol); le 2,5-hexanediol; l'amylèneglycol (ou le 2,4-pentanediol); l'hexylèneglycol (ou le 2-méthyl-2,4-pentanediol) ; l'isoprèneglycol (ou le 3-méthyl-1,3-butanediol); le pinacol (ou le 2,3-diméthyl-2,3-butanediol) ; le 1-méthoxy-2,4-butanediol; le 4-méthoxy-1,2-butanediol; les triols, ramifiés ou non ramifiés, tels que le 1,2,4-butanetriol et le 1,2,6-hexanetriol; les polyéthylène glycol à 4, 6 ou 7 motifs d'éthylène ; le dipropylène glycol.

Selon un mode de réalisation particulier de l'invention, le ou les polyols sont en C₄-C₁₅. De préférence, ils sont choisis parmi l'hexylèneglycol ; le néopentylglycol ; l'isoprèneglycol.

Le ou les polyols sont présents dans la composition conforme à la présente invention dans des proportions généralement comprises entre 0,1 et 40 % en poids, et encore plus particulièrement entre 0,5 et 20 % en poids du poids total de la composition.

La composition tinctoriale de l'invention peut contenir d'autres bases d'oxydation différentes de celles utiles dans la présente invention et conventionnellement utilisées pour la teinture de fibres kératiniques.

La composition de la présente invention peut par exemple comprendre des bases d'oxydation additionnelles choisies parmi les para-phénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis précédemment et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyt)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation additionnelles présentes dans la composition de l'invention sont en général présentes chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est un milieu cosmétique qui généralement contient de l'eau. Il peut également contenir au moins un solvant organique autre que les polyols utiles dans le cadre de l'invention. A titre de solvant organique, on peut par exemple citer les alcanols inférieurs en C₁-C₄, tels que l'éthanol et l'isopropanol et les alcools aromatiques comme l'alcool benzylique, et leurs mélanges.

Les solvants autres que les polyols de l'invention sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 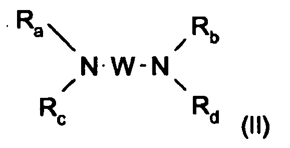 dans laquelle W est un reste propylène éventuellement substitué par un groupement hydroxyle ou un radical alkyle en C₁-C₄ ; Rₐ, R_{b}, Rₑ et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. De préférence, cette coloration est révélée à pH neutre.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus à l'exception de l'agent oxydant et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la coloration d'oxydation des fibres kératiniques, et en particulier des fibres kératiniques humaines telles que les cheveux, d'une composition telle que définie précédemment.

Les dérivés de diamino-N,N-dihydropyrazolone de formule (I) peuvent être obtenus à partir d'intermédiaires et de voies de synthèse décrites dans la littérature et notamment dans les références suivantes : J. Het. Chem., 2001, 38(3), 613-616 , Helvetica Chimica Acta, 1950, 33, 1183-1194, J.Org.Chem., 23, 2029 (1958), J.Am.Chem.Soc., 73, 3240 (1951), J.Am.Chem.Soc., 84, 590 (1962), Justus Liebig Ann.Chem., 686, 134 (1965), Tetrahedron. Lett., 31, 2859-2862 (1973), les brevets US4128425 et US 2841584 et les références citées.

Suivant ces références, les composés de formule (I) ayant les radicaux R₃ et R₄ égaux à des atomes d'hydrogène peuvent être obtenus à partir de la voie de synthèse représentée sur le schéma A ci-dessous :

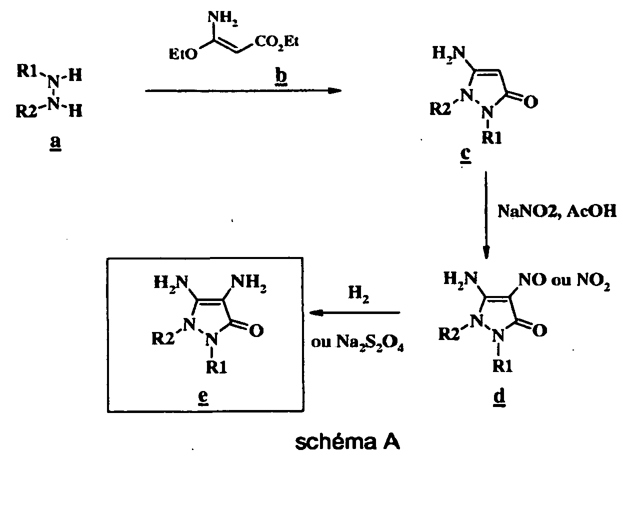

Les composés dont les radicaux R₁ et R₂ représentent simultanément un groupe méthyle et les radicaux R₃ et R₄ des atomes d'hydrogène peuvent être obtenus en s'inspirant de la méthode décrite dans Justus Lieb.Ann.Chem., 686, 134 (1965) (schéma B) :

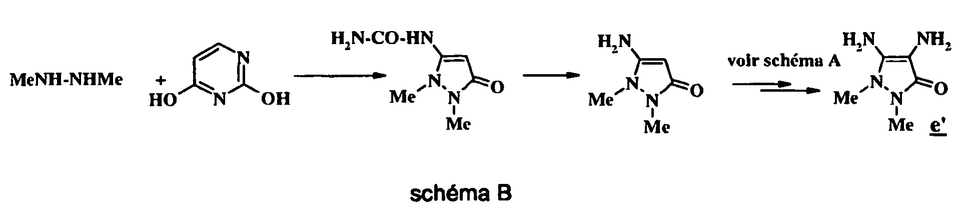

Les composés dont le radical R₁ représente un groupe méthyle, R₂ un radical phényle, et les radicaux R₃ et R₄ des atomes d'hydrogène peuvent être obtenus en s'inspirant de la méthode décrite dans, J.Org.Chem., 23, 2029 (1958), J.Am.Chem.Soc., 73, 3240 (1951) (schéma C) :

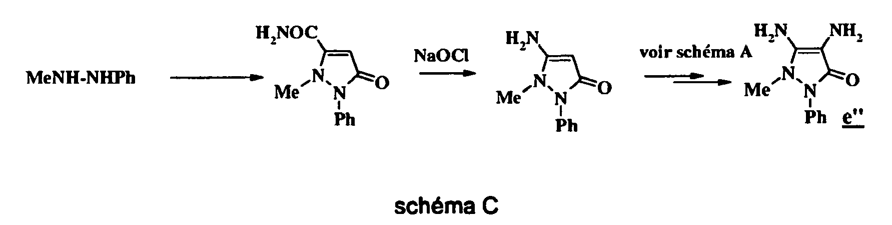

Les composés dont les radicaux R₁ et R₂ forment ensemble un cycle à 5 chaînons et dont les radicaux R₃ et R₄ représentent des atomes d'hydrogène peuvent être obtenus en s'inspirant de la méthode décrite dans J. Het. Chem., 2001, 38(3), 613-616 (schéma D) :

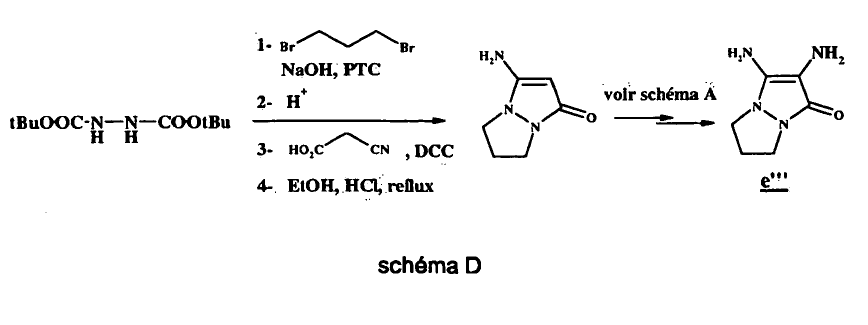

Selon un procédé différent, les composés de formule (I) peuvent être obtenus selon la synthèse illustrée dans le schéma E :

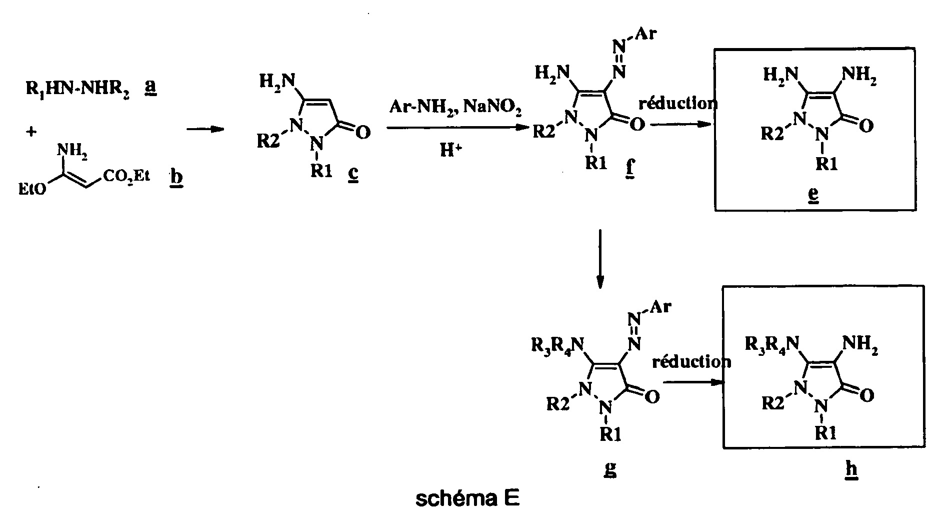

Selon ce procédé, on met en oeuvre les étapes suivantes :
a) étape 1 :on fait réagir un composé **a**

   **R₁HN-NHR₂** **a**

   avec un composé b : 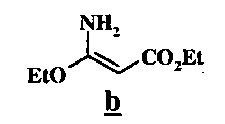 pour obtenir un composé 5-amino-1,2-dihydro-pyrazol-3-one **c** : 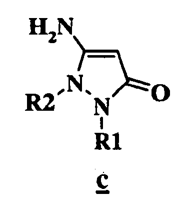
b) étape 2 : on fait réagir le dérivé c ainsi obtenu un sel d'aryldiazonium (Ar-NH₂, NaNO₂, H⁺) pour obtenir un composé azoïque f : 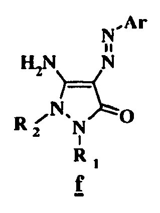
c) étape 3 : on effectue éventuellement une étape de fonctionalisation du groupement amine primaire du composé azoïque résultant f pour obtenir un composé **g** suivant : 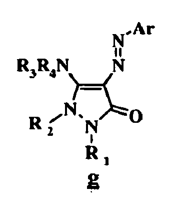
d) étape 4 : on effectue une réaction de réduction du composé azoïque f ou g pour obtenir, respectivement, un composé e ou h aminé :

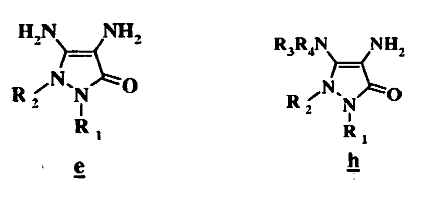

L'étape éventuelle de fonctionalisation du groupement amine primaire en position 5 en amine secondaire et tertiaire NR₃R₄, pour obtenir les composés g, est réalisée selon les méthodes classiques de synthèse organique (halogénure d'alkyle, O-sulfonate d'alkyle, trialkylammonium d'alkyle, amination réductrice, etc... voir par exemple Advanced Organic Chemistry, 3ème édition, 1985, J. March, Willey Interscience*).*

La réduction du groupe azoïque conduit aux composés e et h conformes à l'invention.

L'étape de réduction est réalisée de manière classique, par exemple en effectuant une réaction d'hydrogénation par catalyse hétérogène en présence de Pd / C, Pd(II) / C, Ni / Ra, etc... ou encore en effectuant une réaction de réduction par un métal, par exemple par du zinc, fer, étain, etc. (voir Advanced Organic Chemistry, 3ème édition, J. March, 1985, Willey Interscience et Reduction in organic Chemistry, M . Hudlicky, 1983 , Ellis Horwood Series Chemical Science).

Selon un autre procédé, les dérivés 2,3-diamino-6,7-dihydro-1H,5H-pyrazolo[1,2-a]-pyrazol-1-one et 2,3-diamino-5,6,7,8-tétrahydro-1H,6H-pyridazino[1,2-a]-pyrazol-1-one sont obtenus selon la synthèse illustrée par le schéma F :

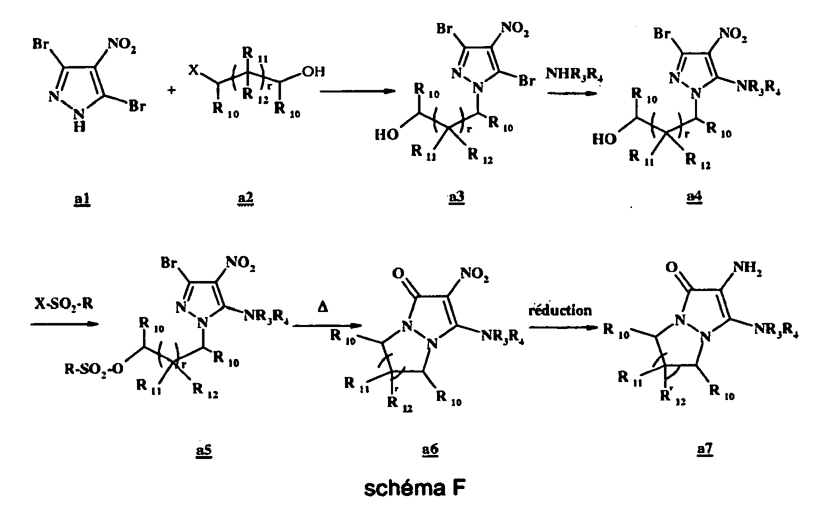

Selon ce procédé, on met en oeuvre les étapes suivantes :
a) étape 1 : on fait réagir un composé a1 suivant : 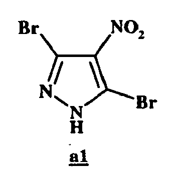 avec un composé a2 : 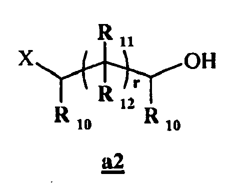 pour obtenir un composé a3 : 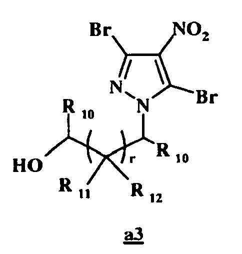 dans lesquelles :
   le radical R₁₀ représente un atome d'hydrogène, un carboxy ; un carboxamido ; un radical alkyle en C₁-C₄ éventuellement substitués par un ou plusieurs radicaux hydroxy, amino, (di)-alkylamino, alcoxy, carboxy, sulfonyle ;
   les radicaux R₁₁ et R₁₂ représentent indépendamment les uns des autres des atomes d'hydrogène, d'halogène ; des radicaux amino ; (di)alkyl(C₁-C₄)amino; hydroxy ; carboxy ; carboxamido ; (C₁-C₂)alcoxy; radical alkyle en C₁-C₄ éventuellement substitués par un ou plusieurs radicaux hydroxy, amino, (di)-alkylamino, alcoxy, carboxy, sulfonyle;
   X représente un atome d'halogène ou un alkyle sulfonate ;
   r est un entier compris entre 1 et 3.
b) étape 2 : on fait réagir le composé a3 avec une amine de formule NHR₃R₄ pour obtenir un composé a4 : 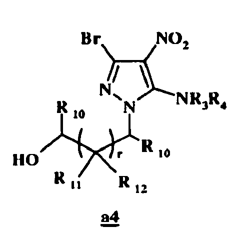
c) étape 3 : on fait réagir le composé a4 avec au moins un halogénure d'alkylsulfonyle, d'arylsulfonyle ou de perfluoroalkylsulfonyle R-O₂S-X₁ (R représente un alkyle, un aryle ou un perfluoroalkyle, X₁ représente un halogène), dans un solvant de point d'ébullition compris entre 60 °C et 190 °C pour obtenir un composé a5 : 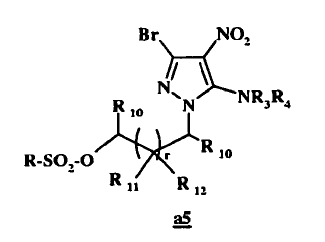
d) étape 4 : le composé a5 résultant est ensuite chauffé dans un solvant de point d'ébullition compris entre 60 °C et 190 °C pour obtenir un composé a6 : 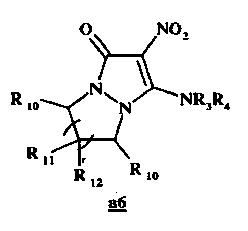
e) étape 5 : Le composé a6 obtenu est réduit pour obtenir le composé a7 de formule ci-dessous (III) :

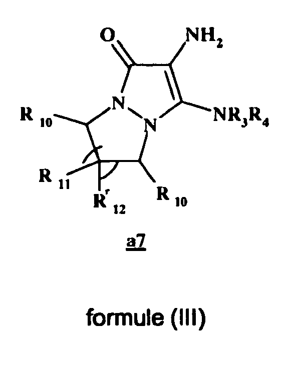

Plus particulièrement, suivant ce procédé, le 3,5-dibromo-4-nitropyrazole a1, obtenu par exemple selon la méthode décrite dans le DE 4234885, réagit avec le réactif a2, de préférence dans un solvant de point d'ébullition compris entre 60 °C et 190 °C. A titre d'exemple, on peut citer le pentanol, le diméthylformamide, la N-méthylpyrrolidine. Plus particulièrement, la réaction est effectuée en présence d'une base organique ou minérale, telle que par exemple le carbonate de sodium, l'hydroxyde de sodium, l'acétate de sodium, ou la triéthylamine. La température du milieu réactionnel est avantageusement maintenue entre 60 °C et 160 °C, de préférence entre 80 °C et 120 °C.

Le 1-hydroxyalkyl-3,5-dibromo-4-nitropyrazole a3 est de préférence isolé par précipitation ou cristallisation après ajout de glace au milieu réactionnel.

Dans l'étape 2, le dérivé a3 est mis en réaction avec une amine NHR₃R₄, de préférence dans un solvant de point d'ébullition compris entre 60 °C et 190 °C, tel que par exemple le butanol, le pentanol, le diméthylformamide. La température est plus particulièrement comprise entre 60 °C et 160 °C, de préférence entre 80 °C et 120 °C. Après consommation des réactifs, le composé 5-amino-4-nitro-3-bromo-1-hydroxyalkylpyrazole a4 est isolé par précipitation ou cristallisation à l'aide d'eau.

Conformément à l'étape 3, le dérivé a5 est obtenu par réaction de l'alcool a4 et d'un halogénure d'alkylsulfonyle, d'arylsulfonyle ou de perfluoroalkylsulfonyle. La réaction a lieu de préférence dans un solvant aprotique tel que par exemple le tétrahydrofurane, le dioxane. La réaction a lieu avantageusement à une température comprise entre -20 °C et 60 °C, de préférence entre 0 °C et 25 °C. De plus, cette étape a lieu en présence d'une base organique ou minérale telle que par exemple le carbonate de potassium, la triéthylamine, la N-méthylmorpholine. Après disparition des réactifs, le composé a5 est isolé par précipitation ou cristallisation dans l'eau.

Le sulfonate a5 obtenu à l'issue de l'étape 3 est mis, dans l'étape 4, en solution ou en dispersion dans un solvant de point d'ébullition compris entre 60 °C et 190 °C, de préférence entre 90 °C et 140 °C. La température du milieu réactionnel est ensuite amenée entre 90 °C et 140 °C, de préférence entre 105 °C et 125 °C jusqu'à consommation totale du sulfonate a5. Après retour à la température ambiante, le composé perhydro-pyrazolo[1,2-a]pyrazol-1-one (r = 1), perhydro-pyridazino[1,2-a]pyrazol-1-one (r = 2) ou perhydro-diazépino[1,2-a]pyrazolone (r = 3) a6 cristallise et est isolé par les méthodes classiques de synthèse organique.

Le composé final a7 conforme à l'invention est obtenu, lors d'une étape 5 par réduction du dérivé nitré a6, les méthodes de réduction utilisées étant par exemple une hydrogénation par catalyse hétérogène en présence de Pd / C, Pd(II) / C, Ni / Ra, etc... ou encore telles une réaction de réduction par un métal, par exemple par du zinc, fer, étain, etc, (voir Advanced Organic Chemistry, 3ème édition, J. March, 1985, Willey Interscience et Reduction in organic Chemistry, M . Hudlicky, 1983, Ellis Horwood Series Chemical Science).

Les exemples qui suivent servent à illustrer l'invention sans toutefois présenter un caractère limitatif.

### EXEMPLES

### Exemple 1 : synthèse du dichlorhydrate de 2,3-diamino-6,7-dihydro-1H,5H-pyrazolo[1,2-a]pyrazol-1-one 5

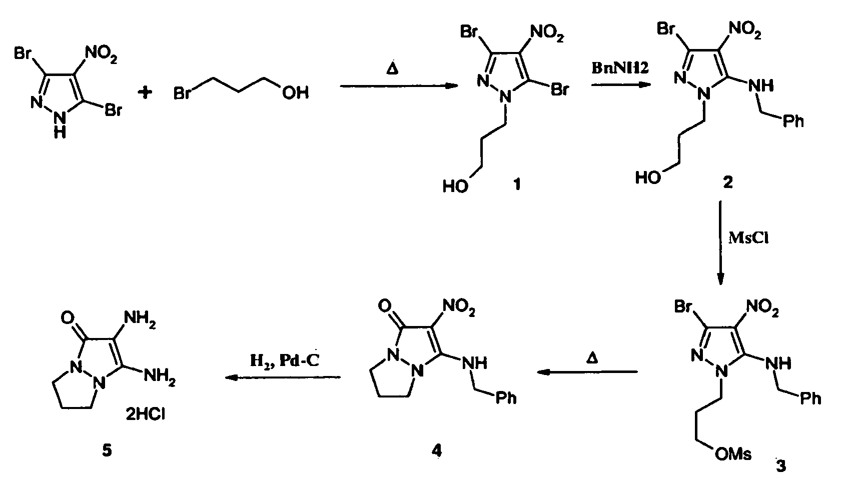

### -Etape 1 : synthèse du 3-(3,5-dibromo-4-nitro-1H-pyrazol-1-yl)propan-1-ol 1

Dans un tricol de 500 ml, on introduit 0,369 mole d'acétate de sodium à une solution de 0,184 mole de dibromonitropyrazole dans 250 ml de N-méthyl pyrrolidone et le milieu réactionnel est porté à 80 °C.

A cette température, on ajoute au goutte à goutte 0,369 mole de 3-bromo propanol. Cette température est maintenue pendant 5 heures.
Après refroidissement à température ambiante, le milieu est versé sur de la glace sous agitation.
Le 3-(3,5-dibromo-4-nitro-1H-pyrazol-1-yl)propan-1-ol 1 précipite. Il est essoré, séché et obtenu avec un rendement de 75 %.

La masse du composé attendu C₆H₇Br₂N₃O₃ est détectée en spectrométrie de masse.

Les analyses RMN (¹H 400 MHz et ¹³C 100,61 MHz DMSO d₆) sont conformes à la structure attendue.

### -Etape 2 : synthèse du 3-[5-(benzylamino)-3-bromo-4-nitro-1H-pyrazol-1-yl]propan-1-ol 2

Dans un tricol de 500 ml contenant 150 ml d'éthanol, on disperse 0,135 mole de 3-(3,5-dibromo-4-nitro-1H-pyrazol-1-yl)propan-1-ol 1, chauffe à 60 °C, puis additionne 0,825 mole de benzylamine en 30 minutes.

Après 6 heures à 60 °C, le milieu réactionnel est refroidi à température ambiante.
Le 3-[5-(benzylamino)-3-bromo-4-nitro-1H-pyrazol-1-yl]propan-1-ol 2 est précipité en versant le milieu réactionnel sur 1 litre de glace sous agitation. Après essorage et séchage sous vide en présence de P₂O₅, le composé 2 est isolé avec un rendement de 90 %.
Les analyses RMN (¹H 400 MHz et ¹³C 100,61 MHz DMSO d₆) sont conformes à la structure attendue.

| Analyse élémentaire : | | | | | |
|---|---|---|---|---|---|
| théorique : | C43,96 | H4,26 | N15,77 | O13,51 | Br22,50 |
| mesuré : | C44,09 | H4,22 | N15,44 | O14,37 | Br21,50 |

### -Etape 3 : synthèse du 3-[5-(benzylamino)-3-bromo-4-nitro-1H-pyrazol-1-yl]propyl méthanesulfonate 3

Dans un tricol de 500 ml contenant 200 ml de THF, on introduit, sous agitation, 0,126 mole de 3-[5-(benzylamino)-3-bromo-4-nitro-1H-pyrazol-1-yl]propan-1-ol 2 et 15,82 ml de triéthylamine. Le mélange obtenu est ensuite refroidi à 5 °C et 0,126 mole de chlorure de mésyle sont coulés en 45 minutes.
Le milieu réactionnel est maintenu à cette température pendant 2 heures, puis le 3-[5-(benzylamino)-3-bromo-4-nitro-1H-pyrazol-1-yl]propylmethanesulfonate 3 est précipité en versant le milieu réactionnel sur 800 ml de la glace.

Après filtration, le solide est lavé abondamment à l'eau et à l'éther diisopropylique. Le séchage est réalisé sous vide en présence de P₂O₅. Le rendement de cette étape est de 94 %.

La masse du composé attendu C₁₄H₁₇BrN₄O₅S est détectée en spectrométrie de masse.

Les analyses RMN (¹H 400 MHz et ¹³C 100,61 MHz DMSO d₆) sont conformes à la structure attendue.

| Analyse élémentaire : | | | | | | |
|---|---|---|---|---|---|---|
| Théorie : | C38,81 | H3,96 | N12,93 | O18,46 | S7,40 | Br18,44 |
| Mesuré : | C39,03 | H3,91 | N12,83 | O18,52 | S7,29 | Br18,26 |

### -Etape 4 : synthèse de la 3-(benzylamino)-2-nitro-6,7-dihydro-1H,5H-pyrazolo[1,2-a]pyrazol-1- one 4

Dans un tricol de 500 ml contenant 300 ml de pentanol, on disperse sous agitation 0,1 mole de 3-[5-(benzylamino)-3-bromo-4-nitro-1H-pyrazol-1-yl]propyl méthanesulfonate 3 et porte le milieu réactionnel à 130 °C pendant 2 heures.

Après refroidissement à température ambiante, le solide formé est essoré sur fritté, lavé à l'éther diisopropylique et séché sous vide en présence de P₂O₅. Le 3-(benzylamino)-2-nitro-6,7-dihydro-1H,5H-pyrazolo[1,2-a]pyrazol-1- one 4 est obtenu avec un rendement de 86 %.
Les analyses RMN (¹H 400 MHz et ¹³C 100,61 MHz DMSO d₆) sont conformes à la structure attendue.
La masse du composé attendu C₆H₁₁N₄O est détectée en spectrométrie de masse.

| Analyse élémentaire : | | | | |
|---|---|---|---|---|
| Théorie : | C56,72 | H5,49 | N20,36 | O17,44 |
| Mesuré : | C56,68 | H5,13 | N20,38 | O17,69 |

### -Etape 5 : synthèse du dichlorhydrate de 2,3-diamino-6,7-dihydro-1H,5H-pyrazolo[1,2-a]pyrazol-1-one 5

Dans un autoclave de 1 litre contenant 800 ml d 'éthanol, on introduit 20 g de 3-(benzylamino)-2-nitro-6,7-dihydro-1H,5H-pyrazolo[1,2-a]pyrazol-1-one 4 et 4 g de palladium sur charbon à 5 %. La réduction est ensuite réalisée sous une pression d'hydrogène de 8 Bars et à une température comprise entre 50 °C et 100 °C (agitation comprise entre 1000 et 2500 tr / min).

Au bout de 4 heures de réaction, il n'a plus consommation d'hydrogène et le milieu est refroidi à 20 °C.

Le catalyseur est éliminé sous azote par filtration, puis de l'éthanol chlorhydrique est ajouté au filtrat. Le produit cristallisé est essoré, lavé à l'éther diisopropylique, puis séché sous vide en présence de P₂O₅. Le dichlorhydrate de 2,3-diamino-6,7-dihydro-1H,5H-pyrazolo[1,2-a]pyrazol-1-one 5 est obtenu avec un rendement de 89 %.

La masse du composé attendu est détectée en spectrométrie de masse.
Les analyses RMN (¹H 400 MHz et ¹³C 100,61 MHz DMSO d₆) sont conformes à la structure attendue.

| Analyse élémentaire : | | | | | |
|---|---|---|---|---|---|
| Théorique : | C31,73 | H5,33 | N24,67 | O7,07 | Cl31,22 |
| Mesuré : | C31,45 | H5,20 | N24,62 | O7,24 | Cl30,86 |

### Exemple 2 : synthèse du dichlorhydrate de 2-amino-3-(éthylamino)-6,7-dihydro-1H,5H-pyrazolo[1,2-a]pyrazol-1-one 9

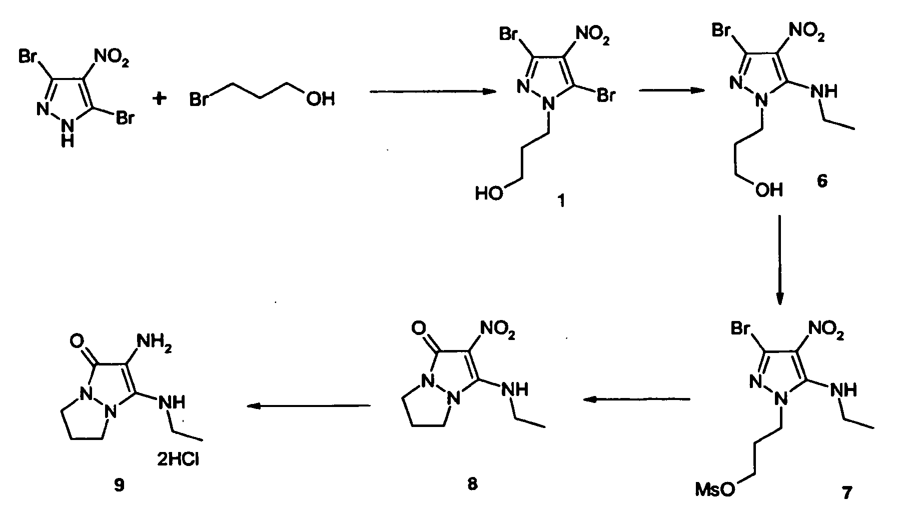

### -Etape 2 :synthèse du 3-[3-bromo-5-(éthylamino)-4-nitro-1H-pyrazol-1-yl]propan-1-ol 6

Dans un tricol, sous agitation, on introduit 15 mmoles de 3-(3,5-dibromo-4-nitro-1H-pyrazol-1-yl)propan-1-ol dans 30 ml d'éthanol. Le milieu homogène est chauffé à 75 °C puis 93 mmoles d'éthylamine sont coulées au goutte à goutte et l'agitation est maintenue quatre heures.

Après refroidissement à température ambiante, le milieu est versé sur de la glace et le 3-[3-bromo-5-(éthylamino)-4-nitro-1H-pyrazol-1-yl]propan-1-ol 6 précipite.

Le solide jaune est essoré, puis lavé abondamment à l'eau et à l'éther diisopropylique. Le séchage est réalisé sous vide en présence de P₂O_{5.} La masse récupérée est de 3,6 g.

Les analyses RMN (¹H 400 MHz et ¹³C 100,61 MHz DMSO d₆) sont conformes à la structure attendue.

La masse du composé attendu C₈H₁₃BrN₄O₃ est détectée en spectrométrie de masse.

### -Etape 3 : synthèse du 3-[5-(éthylamino)-3-bromo-4-nitro-1H-pyrazol-1-yl]propyl methanesulfonate 7

Dans un tricol de 100 ml contenant 30 ml de THF, sous agitation, on introduit 11,2 mmoles de 3-[3-bromo-5-(éthylamino)-4-nitro-1H-pyrazol-1-yl]propan-1-ol 6 et 1,6 ml de triéthylamine. Le mélange homogène orange obtenu est refroidi à 0 °C et 1,44 ml de chlorure de mésyle sont coulés en 20 minutes.

Le milieu réactionnel est maintenu à cette température pendant 2 heures puis le 3-[5-(éthylamino)-3-bromo-4-nitro-1H-pyrazol-1-yl]propylméthanesulfonate 7 est précipité en versant le milieu réactionnel sur 500 ml de glace.

Le solide jaune est essoré, puis lavé abondamment à l'eau et à l'éther diisopropylique ; le séchage est réalisé sous vide en présence de P₂O₅. La masse récupérée est de 3,1 g.

Les analyses RMN (¹H 400 MHz et ¹³C 100,61 MHz DMSO d₆) sont conformes à la structure attendue.

La masse du composé attendu C₉H₁₅BrN₄O₅S est détectée en spectrométrie de masse.

### -Etape 4 : synthèse de la 3-(éthylamino)-2-nitro-6,7-dihydro-1H,5H-pyrazolo[1,2-alpyrazol-1-one 8

Dans un tricol de 50 ml contenant 20 ml de pentanol, on disperse sous agitation 8 mmoles de 3-[5-(éthylamino)-3-bromo-4-nitro-1H-pyrazol-1-yl]propyl méthanesulfonate 7, et porte le milieu réactionnel à 130 °C pendant 2 heures.
Après refroidissement à température ambiante, le solide formé est essoré, puis lavé à l'éther diisopropylique.

Après séchage sous vide en présence de P₂O₅, on obtient 1,46 g de 3-(éthylamino)-2-nitro-6,7-dihydro-1H,5H-pyrazolo[1,2-a]pyrazol-1one 8.

Les analyses RMN (¹H 400 MHz et ¹³C 100,61 MHz DMSO d₆) sont conformes à la structure attendue.

La masse du composé attendu est détectée en spectrométrie de masse.

### -Etape 5 : synthèse du dichlorhydrate de 2-amino-3-(éthylamino)-6,7-dihydro-1H,5H-pyrazolo[1,2-a]pyrazol-1-one 9

Dans un autoclave de 300 ml contenant 200 ml d'éthanol, on introduit 1,45 g de 3-(éthylamino)-2-nitro-6,7-dihydro-1H,5H-pyrazolo[1,2-a]pyrazol-1-one 8 et 300 mg de palladium sur charbon à 5 %. La réduction est réalisée sous une pression d'hydrogène de 8 Bars à une température de 60 °C (agitation de 1700 tr / min).
Au bout de 2 heures de réaction, il n'y a plus consommation d'hydrogène et le milieu est refroidi à 20 °C.

Le catalyseur est éliminé par filtration sous azote et le filtrat est dilué avec 100 ml d'éther isopropylique chlorhydrique.

La solution jaune pale est évaporée à sec puis le solide est repris avec un mélange éthanol / éther isopropylique. Le dichlorhydrate de 2-amino-3-(éthylamino)-6,7-dihydro-1H,5H-pyrazolo[1,2-a]pyrazol-1-one 9 précipite ; il est essoré et après séchage sous vide en présence de P₂O₅, on récupère 1,18 g de dichlorhydrate de 2-amino-3-(éthylamino)-6,7-dihydro-1H,5H-pyrazolo[1,2-a]pyrazol-1-one 9.

Les analyses RMN (¹H 400 MHz et ¹³C 100,61 MHz DMSO d₆) sont conformes à la structure attendue.

La masse du composé attendu C₈H₁₄N₄O est détectée en spectrométrie de masse.

### Exemple 3 : dichlorhydrate de 2-amino-3-(isopropylamino)-6,7-dihydro-1H,5H-pyrazolo[1,2-a]pyrazol-1-one 13

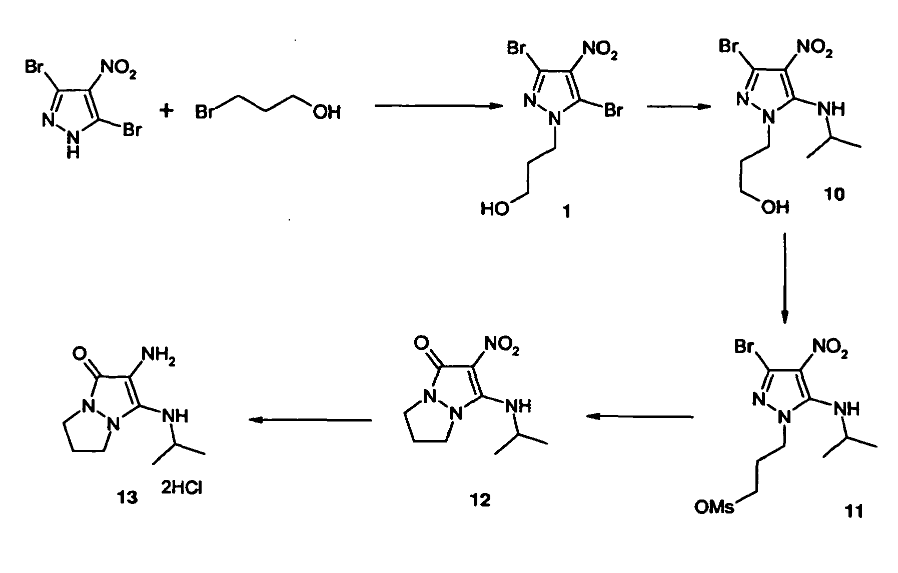

### -Etape 2 : 3-[3-bromo-5-(isopropylamino)-4-nitro-1H-pyrazol-1-yl]propan-1-ol 10

Dans un tricol, sous agitation, on introduit 15 mmoles de 3-(3,5-dibromo-4-nitro-1H-pyrazol-1-yl)propan-1-ol dans 30 ml d'éthanol. Le milieu homogène est chauffé à 75 °C, puis 93 mmoles d'isopropylamine sont coulées au goutte à goutte tout en maintenant l'agitation quatre heures.

Après refroidissement à température ambiante, le milieu est versé sur de la glace, puis neutralisé à l'acide chlorhydrique. On extrait le 3-[3-bromo-5-(isopropylamino)-4-nitro-1H-pyrazol-1-yl]propan-1-ol 10 au dichlorométhane.

Après séchage de la phase organique sur sulfate de sodium et élimination du solvant par évaporation sous vide, on obtient 4,37 g de 3-[3-bromo-5-(isopropylamino)-4-nitro-1H-pyrazol-1-yl]propan-1-ol 10.

Les analyses RMN (¹H 400 MHz et ¹³C 100,61 MHz DMSO d₆) sont conformes à la structure attendue.

La masse du composé attendu C₉H₁₅BrN₄O₃ est détectée en spectrométrie de masse.

### -Etape 3: synthèse du 3-[5-(isopropylamino)-3-bromo-4-nitro-1H-pyrazol-1-yl]propyl méthanesulfonate 11

Dans un tricol de 50 ml contenant 20 ml de THF, on introduit, sous agitation, 13,7 mmoles de 3-[3-bromo-5-(isopropylamino)-4-nitro-1H-pyrazol-1-yl]propan-1-ol 10 et 1,94 ml de triéthylamine. Le mélange homogène orange ainsi obtenu est refroidi à 0 °C et 1,76 ml de chlorure de mésyle sont coulés en 20 minutes.

Le milieu réactionnel est maintenu à cette température pendant 2 heures, puis le 3-[5-(éthylamino)-3-bromo-4-nitro-1H-pyrazol-1-yl]propylméthanesulfonate 11 est précipité en versant le milieu réactionnel sur 500 ml de glace.

Le solide jaune est essoré, puis lavé abondamment à l'eau et à l'éther de pétrole, le séchage est réalisé sous vide en présence de P₂O₅. La masse récupérée est de 4,2 g.

Les analyses RMN (¹H 400 MHz et ¹³C 100,61 MHz DMSO d₆) sont conformes à la structure attendue.

La masse du composé attendu est détectée en spectrométrie de masse.

### -Etape 4: synthèse de la 3-(isopropylamino)-2-nitro-6,7-dihydro-1H,5H-pyrazolo[1,2-a]pyrazol-1-one 12

Dans un tricol de 50 ml, on disperse, sous agitation, 10 mmoles de 3-[5-(isopropylamino)-3-bromo-4-nitro-1H-pyrazol-1-yl]propylméthanesulfonate 11 dans 20 ml de pentanol et chauffe à 130 °C pendant 2 heures.

Après refroidissement à température ambiante, le solide obtenu est essoré sur fritté, et lavé à l'éther diisopropylique.

Après séchage sous vide en présence de P₂O₅, 1,71 g de 3-(isopropylamino)-2-nitro-6,7-dihydro-1H,5H-pyrazolo[1,2-a]pyrazol-1one 12 sont obtenus.

Les analyses RMN (¹H 400 MHz et ¹³C 100,61 MHz DMSO d₆) sont conformes à la structure attendue.

La masse du composé attendu C₉H₁₄N₄O₃ est détectée en spectrométrie de masse.

### -Etape 5: synthèse du dichlorhydrate de 2-amino-3-(isopropylamino)-6,7-dihydro-1H,5H-pyrazolo[1,2-a]pyrazol-1-one 13

Dans un autoclave de 300 ml contenant 200 ml d 'éthanol, on introduit 1,70 g de 3-(isopropylaminoamino)-2-nitro-6,7-dihydro-1H,5H-pyrazolo[1,2-a]pyrazol-1-one 12 et 300 mg de palladium sur charbon à 5 %. La réduction est réalisée sous une température de 60 °C et sous une pression d'hydrogène de 6 Bars (agitation de 2000 tr / min).

Au bout de 2 heures de réaction, il n'a plus consommation d'hydrogène et le milieu est refroidi à 20 °C.
Le catalyseur est éliminé par filtration sous azote après refroidissement à température ambiante et de l'éther isopropylique chlorhydrique est additionné.

La solution jaune pale est évaporée à sec, puis le solide est repris avec 50 ml d'éther diisopropylique saturé en acide chlorhydrique, le précipité est récupéré par essorage. Après séchage sous vide en présence de P₂O₅, 1,5 g de dichlorhydrate de 2-amino-3-(isopropylamino)-6,7-dihydro-1H,5H-pyrazolo[1,2-a]pyrazol-1-one 13 sont isolés.

Les analyses RMN (¹H 400 MHz et ¹³C 100,61 MHz DMSO d₆) sont conformes à la structure attendue.

La masse du composé attendu C₉H₁₆N₄O est détectée en spectrométrie de masse.

### Exemple 4 : dichlorhvdrate de 2-amino-3-(pyrrolidin-1-yl)-6,7-dihydro-1H,5H-pyrazolo[1.2-a]pyrazol-1-one 17

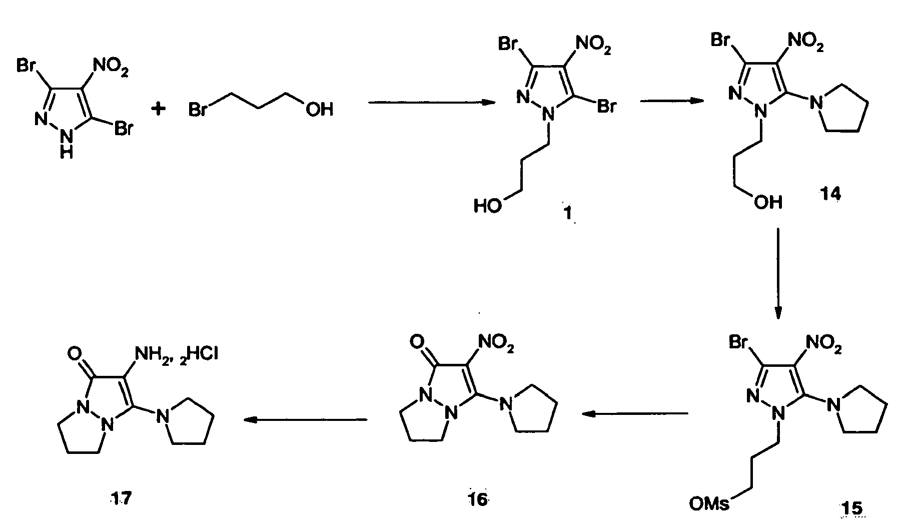

### -Etape 2 : 3-(3-bromo-4-nitro-5-(pyrrolidin-1-yl)-1H-pyrazol-1-yl)propan-1-ol 14

Dans un tricol, sous agitation, on introduit 15 mmoles de 3-(3,5-dibromo-4-nitro-1H-pyrazol-1-yl)propan-1-ol dans 20 ml d'isopropanol. Le milieu homogène est chauffé à 75 °C puis 90 mmoles de pyrrolidine sont coulées au goutte à goutte et l'agitation est maintenue deux heures.

Après refroidissement à température ambiante, le milieu est versé sur de la glace et neutralisé à l'acide chlorhydrique. On extrait le 3-(3-bromo-4-nitro-5-(pyrrolidin-1-yl)-1H-pyrazol-1-yl)propan-1-ol 14 par du dichlorométhane.

Après séchage de la phase organique sur sulfate de sodium et distillation du solvant par évaporation sous vide, on obtient 4,8 g de 3-(3-bromo-4-nitro-5-(pyrrolidin-1-yl)-1H-pyrazol-1-yl)propan-1-ol 14.

Les analyses RMN (¹H 400 MHz et ¹³C 100,61 MHz DMSO d₆) sont conformes à la structure attendue.

La masse du composé attendu C₁₀H₁₇BrN₄O est détectée en spectrométrie de masse.

### -Etape 3 : synthèse du 3-(3-bromo-4-nitro-5-(pyrrolidin-1-yl)-1H-pyrazol-1-yl)propylméthanesulfonate 15

Dans un tricol de 100 ml contenant 50 ml de THF, on introduit, sous agitation, 30 mmoles 3-(3-bromo-4-nitro-5-(pyrrolidin-1-yl)-1H-pyrazol-1-yl)propan-1-ol 14 et 4,25 ml de triéthylamine. Le mélange homogène orange obtenu est refroidi à 0 °C et 2,32 ml de chlorure de mésyle sont coulés en 20 minutes.

Le milieu réactionnel est maintenu à cette température pendant 2 heures puis le 3-(3-bromo-4-nitro-5-(pyrrolidin-1-yl)-1H-pyrazol-1-yl)propylméthanesulfonate 15 est précipité en versant le milieu réactionnel sur de la glace.
Le solide est essoré, puis séché sous vide en présence de P₂O₅. La masse récupérée est de 9,3 g.

Les analyses RMN (¹H 400 MHz et ¹³C 100,61 MHz DMSO d₆) sont conformes à la structure attendue.

La masse du composé attendu C₁₁H₁₉BrN₄O₃S est détectée en spectrométrie de masse.

### -Etape 4 : synthèse 2-nitro-3-(pyrrolidin-1-yl)-6,7-dihydro-1H,5H-pyrazolo[1,2-a]pyrazol-1-one 16

Dans un tricol de 250 ml, on introduit, sous agitation, 22,5 mmoles de 3-(3-bromo-4-nitro-5-(pyrrolidin-1-yl)-1H-pyrazol-1-yl)propylméthanesulfonate 15 dans 100 ml de pentanol. Le milieu ainsi obtenu est porté à 130 °C pendant 2 heures.

Après refroidissement à température ambiante, le 2-nitro-3-(pyrrolidin-1-yl)-6,7-dihydro-1H,5H-pyrazolo[1,2-a]pyrazol-1-one 16 est extrait au dichlorométhane.

Après séchage de la phase organique sur sulfate de sodium et distillation du solvant sous vide, on obtient 1,2 g de 2-nitro-3-pyrrolidin-1-yl-6,7-dihydro-1H,5H-pyrazolo[1,2-a]pyrazol-1-one 16.

Les analyses RMN (¹H 400 MHz et ¹³C 100,61 MHz DMSO d₆) sont conformes à la structure attendue.

La masse du composé attendu C₁₀H₁₄N₄O₃ est détectée en spectrométrie de masse.

### -Etape 5 : synthèse du dichlorhydrate de 2-amino-3-(pyrrolidin-1-yl)-6,7-dihydro-1H,5H-pyrazolo[1,2-a]pyrazol-1-one 17

Dans un autoclave de 300 ml contenant 200 ml d 'éthanol, on introduit 1,1 g de 2-nitro-3-(pyrrolidin-1-yl)-6,7-dihydro-1H,5H-pyrazolo[1,2-a]pyrazol-1-one 16 et 300 mg de palladium sur charbon à 5 %. La réduction est réalisée sous une agitation de 2000 tr / min, sous une température de 60 °C et sous une pression d'hydrogène de 6 Bars.

Au bout de 2 heures de réaction, il n'a plus consommation d'hydrogène et le milieu est refroidit à 20 °C.

Le catalyseur est éliminé par filtration sous azote après refroidissement à température ambiante et de l'éther isopropylique chlorhydrique est additionné.

La solution jaune pale est évaporée à sec, puis le solide est repris avec 50 ml d'éther diisopropylique saturé en acide chlorhydrique, le précipité est récupéré par essorage. Après séchage sous vide en présence de P₂O₅, on obtient 1,5 g de dichlorhydrate de 2-amino-3-(pyrrolidin-1-yl)-6,7-dihydro-1H,5H-pyrazolo[1,2-a]pyrazol-1-one 5 17.

Les analyses RMN (¹H 400 MHz et ¹³C 100,61 MHz DMSO d₆) sont conformes à la structure attendue.

La masse du composé attendu C₁₀H₁₆N₄O est détectée en spectrométrie de masse.

### Exemple 5 : Synthèse du diméthanesulfonate de 2,3-diamino-6,7-dihydro-1H,5H-pyrazolo[1,2-a]pyrazol-1-one

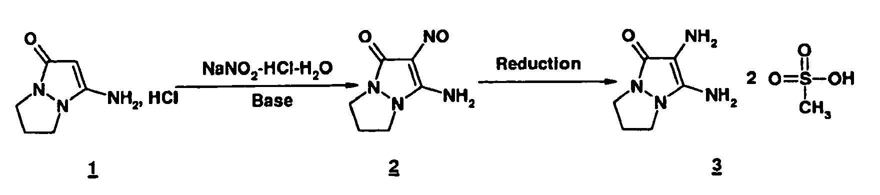

### Synthèse du 3-amino-2-nitroso-6,7-dihydro-1H,5H-pyrazolo[1,2-a]pyrazol-1-one : 2

Dans un tricol de 500 ml, on dissout, sous agitation, à température ambiante, 43 g (0,245 mole) de chlorhydrate de 3-amino-6,7-dihydro-1H,5H-pyrazolo[1,2-a]pyrazol-1-one dans un mélange de 180 ml d'eau et 35 ml d'acide chlorhydrique à 35 %.

On refroidit à 0 °C et ajoute goutte à goutte, en 30 minutes, une solution de 17,3 g de nitrite de sodium (0,25 mole) dans 20 ml d'eau. La température du milieu réactionnel est maintenue entre 0 et +5 °C pendant toute la durée de l'addition et pendant une heure après la fin de l'addition.

Le milieu réactionnel est amené à pH 8 par addition de soude, sous agitation, tout en maintenant la température entre 0 et 5 °C. La 3-amino-2-nitroso-6,7-dihydro-1H,5H-pyrazolo[1,2-a]pyrazol-1-one 2 précipite sous la forme d'un solide orangé rouge qui est filtré sur verre fritté n° 4, empâté dans le minimum de 2-propanol, lavé à l'éther diisopropylique et séché sous vide en présence de pentaoxyde de phosphore. On obtient ainsi 35 g de produit rouge orangé (rendement : 85 %).

Les spectres de RMN (1H 400 MHz et 13C 100,61 MHz DMSO d₆) et de masse sont conformes à la structure attendue 2.

### Synthèse du diméthanesulfonate de 2,3-diamino-6,7-dihydro-1H,5H-pyrazolo[1,2-a]pvrazol-1-one : 3

Dans un autoclave de 1 litre, on introduit 33,6 g (0,2 mole) de 3-amino-2-nitroso-6,7-dihydro-1H,5H-pyrazolo[1,2-a]pyrazol-1-one 2, 500 ml d'éthanol et 6 g de palladium sur charbon à 5 % contenant 50 % d'eau.
Le milieu est purgé 3 fois à l'azote puis 3 fois à l'hydrogène et la température du mélange est portée à 40 °C.
La réduction est réalisée en deux heures sous une pression de 8 bars. Cette réduction est exothermique et la température atteint d'elle-même 70 °C.
On laisse la température redescendre à 50 °C puis le catalyseur est filtré sur un filtre presse sous un courant d'azote.
Le filtrat est coulé dans un mélange de 50 ml d'éthanol et 40 ml d'acide méthane sulfonique, en refroidissant à 0 °C. Le diméthanesulfonate de 2,3-diamino-6,7-dihydro-1H,5H-pyrazolo[1,2-a]pyrazol-1-one 3 cristallise sous la forme d'un solide jaune pâle qui est essoré sur verre fritté n° 4, lavé à l'éther düsopropylique puis à l'éther de pétrole et enfin séché sous vide en présence de pentaoxyde de phosphore. On obtient ainsi 43 g de solide jaune pâle (rendement : 65 %).
Les spectres de RMN (¹H 400 MHz et ¹³C 100,61 MHz DMSO d₆) et de masse sont conformes à la structure attendue 3.

| Analyse élémentaire : | | | | | |
|---|---|---|---|---|---|
| Théorie : | C27,74 | H5,23 | N16,17 | O32,33 | S18,51 |
| Mesuré : | C27,16 | H5,22 | N15,63 | O32,81 | S18,64 |

### Exemple 6 : synthèse du chlorhydrate de 2,3-diamino-5,6,7,8-tetrahydro-1H-pyrazolo[1,2-a]pyridazin-1-one.

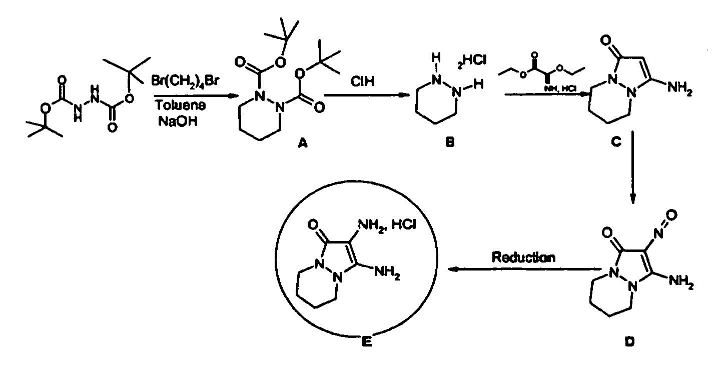

### Synthèse du di-tert-butyl tetrahydropyridazine-1,2-dicarboxylate : A

Dans un tricol de 250 ml équipé d'un réfrigérant, d'un thermomètre et d'une ampoule de coulée, on introduit sous agitation mécanique 50 ml de toluène, 5 g (21,5 mmoles) de N,N'-di-tert-butoxycarbonylhydrazide, 680 mg de bromure de tétraéthylammonium et 25 ml de soude à 50 %.
Le milieu hétérogène est chauffé à 100 °C puis on ajoute goutte à goutte en 15 minutes le 1,4-dibromobutane.
Le milieu réactionnel est chauffé à 100 °C pendant 3 jours. Après refroidissement, on ajoute 100 ml d'acétate d'éthyle et on transfère dans une ampoule à décanter. La phase organique est lavée avec 4 fois 70 ml de solution aqueuse saturée en carbonate de sodium, puis avec 4 x 70 ml d'eau et enfin avec 4 x 70ml d'eau salée. La phase organique est séchée sur sulfate de sodium et le solvant est évaporé sous vide. On obtient ainsi une huile incolore qui cristallise en un solide blanc.
On récupère une masse de 6,1 g (rendement : 99 %).
Les spectres RMN (¹H 400 MHz et ¹³C 100,61 MHz DMSO d₆) et de masse sont conformes à la structure attendue A.

### Synthèse du dichlorhydrate d'hexahydropyridazine : B

Dans un tricol de 100 ml équipé d'un réfrigérant et d'un thermomètre, on introduit sous agitation mécanique 5,9 g du composé A dans 50 ml de mélange 3 / 1 de dioxanne et d'acide chlorhydrique à 35 %.
La solution incolore obtenue est agitée à température ambiante pendant 3 heures puis le milieu réactionnel est dilué par de l'éther diisopropylique. Les solvants sont évaporés sous vide. Le résidu pâteux obtenu est repris par un mélange éther / éthanol. Après filtration du solide et séchage sous vide, on obtient 1,39 g de solide blanc.

Les spectres de RMN (¹H 400 MHz et ¹³C 100,61 MHz DMSO d₆) et de masse sont conformes à la structure attendue B.

### Synthèse du 3-amino-5,6,7,8-tetrahydro-1H-pyrazolo[1,2-a]pyridazin-1-one : C

Dans un tricol de 25 ml équipé d'un réfrigérant et d'un thermomètre, on introduit sous agitation mécanique 7,5 ml d'éthanol, 1,5 ml de triéthylamine et 0,73 ml d'acide 3-amino-3-ethoxyacrylique. On ajoute ensuite 500 mg de dichlorhydrate d'hexahydropyridazine (composé B) et on agite pendant 3 heures à température ambiante.
On filtre l'insoluble et distille le solvant sous vide. Le solide est repris par le minimum d'eau, filtré et séché sous vide. On obtient ainsi 0,9 g de poudre légèrement jaune.

Les spectres de RMN (¹H 400 MHz et ¹³C 100,61 MHz DMSO d₆) et de masse sont conformes à la structure attendue C.

### Synthèse du 3-amino-2-nitroso-5,6,7,8-tetrahydro-1H-pyrazolo[1,2-a]pyridazin-1-one : D

Dans un tricol de 50 ml équipé d'un réfrigérant et d'un thermomètre, on introduit sous agitation mécanique 20 ml d'acide chlorhydrique à 35 % et 1 g de 3-amino-5,6,7,8-tetrahydro-1H-pyrazolo[1,2-a]pyridazin-1-one (composé C).
On refroidit à 0 °C et on coule une solution de 675 mg nitrite de sodium dans 5 ml d'eau en maintenant cette température. La couleur du mélange réactionnel vire du jaune à l'orange et un précipité commence à se former.
En 30 minutes, la réaction est terminée et le solide orange est filtré sur verre fritté n° 4, lavé à l'eau puis séché sous vide. Le rendement est de 78,3 %.
Les spectres de RMN (¹H 400 MHz et ¹³C 100,61 MHz DMSO d₆) et de masse sont conformes à la structure attendue D.

### Synthèse du chlorhydrate de 2,3-diamino-5,6,7,8-tetrahydro-1H-pyrazolo[1.2-a]pyridazin-1-one : E

Dans un autoclave de 300 ml contenant 250 ml d 'éthanol, on introduit 1,3 g de 3-amino-2-nitroso-5,6,7,8-tetrahydro-1H-pyrazolo[1,2-a]pyridazin-1-one (composé D) et 250 mg de palladium sur charbon à 5 %. La réduction est réalisée sous une agitation de 2000 tr / min, à une température de 60 °C et sous une pression d'hydrogène de 6 bars.
Au bout de 2 heures de réaction, il n'y a plus de consommation d'hydrogène et le milieu est refroidi à 20 °C.
Le catalyseur est éliminé par filtration sous azote après refroidissement à température ambiante et la solution est versée sur 75 ml de dioxanne chlorhydrique.

La solution ainsi obtenue est évaporée jusqu'à obtention d'une poudre légèrement jaune que l'on reprend dans de l'éther diisopropylique.
Le solide est récupéré par filtration. Après séchage sous vide en présence de pentaoxyde de phosphore, on obtient 1,1 g de dichlorhydrate de 2,3-diamino-5,6,7,8-tetrahydro-1H-pyrazolo[1,2-a]pyridazin-1-one.
Les spectres de RMN (¹H 400 MHz et ¹³C 100,61 MHz DMSO d₆) et de masse sont conformes à la structure attendue E.

### Exemple 7: Synthèse du chlorhydrate de 4-Amino-1,2-diethyl-5-pyrrolidin-1-yl-1,2-dihydro-pyrazol-3-one

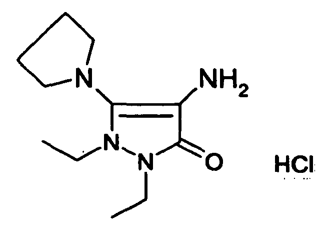

### Etape 1 : Synthèse de la 1, 2 ― diéthylpyrazolidine-3,5-dione

Dans un tricol de 3000 ml muni d'un thermomètre et sous agitation magnétique, on introduit successivement sous atmosphère d'azote 100 g du dichlorhydrate de diéthylhydrazine (0,63 moles) dans 1000 ml de dichlorométhane, 85,3 g d'acide malonique (0,82 moles ; 1,3 éq.), 196 g de hydroxybenzotriazole (1,45 moles ; 2,3 éq.), 278 g de 1-(3-diméthylaminopropyl)-3-ethyl-carbodümide, hydrochlorure (EDCl ; 1,45 moles ; 2,3 éq.).

Le milieu réactionnel est ensuite refroidi entre 0 °C et 5 °C. On y additionne alors lentement 407 g de N, N-diisopropylethylamine (3,14 moles ; 520 ml : 5 éq.). A la fin de l'addition, le milieu réactionnel devenu homogène est laissé sous agitation à température ambiante. Après une nuit à température ambiante, la réaction est terminée.

Le milieu réactionnel est lavé par trois fois 600 ml d'eau permutée. La phase organique est séchée sur sulfate de sodium, filtrée et concentrée sous vide pour fournir 46g de produit brut. La pyrazolidine-dione étant soluble en milieu aqueux, la phase aqueuse est donc concentrée à sec puis reprise par 800 ml d'une solution d'acide chlorhydrique 1 N. Le précipité formé est filtré et la phase aqueuse est extraite par trois fois 1300 ml de dichlorométhane. Les phases organiques rassemblées sont séchées sur sulfate de sodium, filtrées et concentrées sous vide pour fournir 67,5 g de poduit brut.

On obtient ainsi la 1,2-diéthylpyrazolidine-3,5-dione sous forme de solide jaune avec un rendement de 40 % (39,5 g).

### Etape 2 : Synthèse de la 1,2-diéthyl-3-chloro-5-pyrazolone

Dans un tricol de 500 ml équipé d'un réfrigérant et d'une agitation magnétique, sont introduits, sous atmosphère d'azote, 30 g de 1,2 g de 1,2-diéthylpyrazolidine-3,5-dione (0,19 moles) en solution dans 200 ml de toluène et 35,8 ml d'oxyde de trichlorophosphine (258,9 g ; 0,38 moles ; 2 éq.).

Le milieu réactionnel est porté au reflux du toluène et la réaction est suivie par CCM (dichlorométhane / méthanol 95 / 5). Le milieu réactionnel initialement sous forme de pâte, s'homogénéise dès le reflux puis devient biphasique.

Après une heure de reflux, la réaction est hydrolysée à 0 °C par addition très lente de 100 ml d'eau permutée. Après décantation, la phase toluène est séparée de la phase aqueuse. La phase aqueuse est lavée par 50 ml de toluène puis amenée à pH 12 par 184 ml d'une solution de soude à 35 %. On observe la formation d'un précipité. La phase aqueuse est portée à 100 °C pendant 10 minutes et le précipité se solubilise. Le milieu réactionnel présente alors deux phases. La phase supérieure de coloration brune est séparée après décantation à chaud. Cette phase supérieure est solubilisée par 200 ml de dichlorométhane, lavée par une fois 50 ml d'eau permutée, séchée sur sulfate de sodium et concentrée sous vide pour fournir 20,5 g d'une huile brune.

Un précipité se forme dans la phase aqueuse inférieure lors du retour à la température ambiante. Après filtration sur fritté, le précipité est rincé à l'eau et le filtrat est extrait par trois fois 300 ml de dichlorométhane. La phase dichlorométhane est séchée sur sulfate de sodium et concentrée sous vide pour fournir 5,5 g de cristaux bruns.

L'huile et les cristaux bruns sont rassemblés, greffés sur silice et chromatographiés sur gel de silice (40-60 µm ; 2000 g) par un gradient d'élution :
1) Dichlorométhane 100 (13 litres)
2) Dichlorométhane / MeOH 99,5 / 0,5 (0,8 litres)
3) Dichlorométhane / MeOH 99 / 1 (8 litres) produit attendu + 15 % impureté m = 6,6 g
4) Dichlorométhane / MeOH 98,5 / 1,5 (35 litres) produit attendu (14,7 g)

La 1,2-diéthyl-3-chloro-5-pyrazolone est ainsi obtenue sous la forme de cristaux jaune avec un rendement de 44%.

### Etape 3 : Synthèse du 1,2-Diethyl-5-pyrrolidin-1-yl-1,2-dihydro-pyrazol-3-one

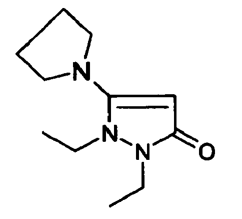

Dans un réacteur 2,5 ml du micro-onde Biotage initiator, on introduit 1 g de 5-chloro-1,2-diéthyl-1,2-dihydro-pyrazol-3-one (5,7.10⁻⁴ moles), auquel on ajoute 2 ml de pyrrolidine (4,2 éq.).

Conditions opératoires : micro-onde à puissance maximum θ = 120 °C pendant 17 min.

Après 17 minutes, la réaction est terminée (suivi CCM éluant : 90 / 10 CH₂Cl₂ / MeOH).

5 ml d'eau déminéralisée sont alors ajoutés au milieu réactionnel, puis l'ensemble est transféré en ampoule à décanter. La phase aqueuse est extraite avec quatre fois 10 ml de dichlorométhane. Les phases organiques sont ensuite réunies et séchées sur du sulfate de sodium anhydre, puis filtrées et évaporées à sec. 1,2 grammes d'une huile orangée brune sont obtenus avec un rendement de 100 %.
RMN (¹H 400 MHz DMSO d₆)
0,81 (1t, 3H), 0,89 (1t, 3H), 1,88 (1m, 1H), 3,22 (1m, 4H), 3,4 (1m, 4H), 4,4 (1s, 1H)
Masse : Analyse faite en OpenLynx (FIA/MS).

La masse principalement détectée est en accord avec la structure attendue : M = 20

### Etape 4: Synthèse du 1,2-diéthyl-4-nitroso-5-pyrrolidin-1-yl-1,2-dihydro-3H-pyrazol-3-one

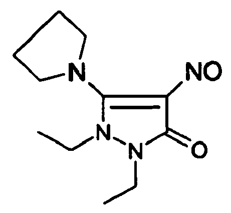

Dans un tricol de 25 ml tout équipé, on introduit 1,2 g de 1,2-diéthyl-5-pyrrolidin-1-yl-1,2-dihydro-pyrazol-3-one que l'on solubilise dans un mélange composé de 0,84 ml d'acide chlorhydrique 37 % et de 4 ml d'eau déminéralisée.
Le milieu réactionnel est refroidit entre 0 °C et 5 °C, à l'aide d'un bain d'eau glacée.
Une solution composée de 400 mg de nitrite de sodium (5,7.10⁻⁴ moles) solubilisés dans 0,6 ml d'eau déminéralisée, est alors ajoutée au goutte à goutte.
Le milieu réactionnel vire instantanément au rouge vif dès l'ajout de la première goutte du mélange précédent.

Après une heure, la réaction est terminée.
Le pH est ajusté à environ 7-8 à l'aide d'une solution de soude à 30 %, puis le milieu réactionnel est transféré en ampoule à décanter. La phase aqueuse est extraite avec 4 fois 10 ml de dichloromethane. Les phases organiques sont réunies et séchées sur sulfate de sodium anhydre puis évaporées à sec. 1,2 grammes d'une poudre bleu turquoise sont obtenus avec un rendement de 89,6 %.

Les spectres de RMN (¹H 400 MHz DMSO d₆) et de masse sont conformes à la structure attendue.
RMN (¹H 400 MHz DMSO d₆)
0,94 (1t, 3H), 1 (1t, 3H), 2,05 (1m, 4H), 3,51 (1q, 4H), 3,76 (1q, 4H), 3,94 (1m, 4H)
Analyse faite en OpenLynx (FIA/MS).
La masse principalement détectée est en accord avec la structure attendue. M = 238.

### Etape 5 : Synthèse du chlorhydrate de 4-Amino-1,2-diéthyl-5-pyrrolidin-1-yl-1,2-dihydro-pyrazol-3-one :

Dans un tricol de 500 ml tout équipé, on introduit 4 grammes de zinc en poudre (0,06 moles) dans 300 ml d'éthanol absolu auxquels on ajoute 1 ml d'acide acétique.
Le milieu réactionnel est chauffé à 40 °C, puis 1,15 g (4,8.10⁻³ moles) de 1,2-diéthyl-4-nitroso-5-pyrrolidin-1-yl-1,2-dihydro-3H-pyrazol-3-one sont introduits par petites spatules. 4 ml d'acide acétique sont enfin introduits ml par ml et le milieu est porté au reflux. Le milieu est totalement soluble et incolore. Après 30 minutes, la réaction est terminée sur CCM selon l'éluant acétate d'éthyle / MeOH 90/10.

Le milieu réactionnel est refroidi puis filtré sur fritté contenant un lit de cellite 545. Les eaux mères sont filtrées dans un ballon contenant 2,5 ml d'isopropanol chlorhydrique 5 N refroidi. Le tout est ensuite évaporé à sec. Le produit obtenu est une poudre rose conforme par RMN et Masse.
RMN (¹H 400 MHz DMSO d₆)
0,79 (1t, 3H), 0,96 (1t, 3H), 1,87 (1m, 4H), 3,49 (1q, 2H), 3,59 (1m, 6H)
Analyse FIA/MS réalisée via OpenLynx.
Les ions quasi moléculaires [M+H]+, [M+Na]+, [2M+H]+, [2M+Na]+ de la base attendue C₁₁H₂₀N₄O sont principalement détectés.

En reproduisant les étapes précédentes avec les réactifs appropriés, on peut obtenir le 4-amino-5-[3-(diméthylamino)pyrrolidin-1-yl]-1,2-diéthyl-1,2-dihydro-3H-pyrazol-3-one hydrochloride.

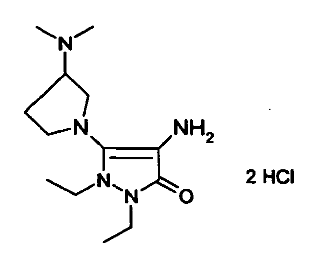

### EXEMPLES DE TEINTURE

### Exemple 1 :

La composition 1 suivante a été réalisée :

| | |
|---|---|
| Mélange d'alcools linéaires en C₁₈ à C₂₄ [C₁₈/C₂₀/C₂₂/C₂₄: 7/58/30/6, teneur en alcools > 95 %] | 3 g |
| Mélange d'alcools linéaires en C₁₈ à C₂₄ [C₁₈/C₂₀/C₂₂/C₂₄: 7/58/30/6, teneur en alcools > 95 %] oxyéthylénés (30 OE) | 1 g |
| Alcool stéarylique oxyéthyléné (2 OE) | 4,5 g |
| Acide stéarylique oxyéthyléné (21 OE) | 1,75 g |
| Acide polyacrylique réticulé : | |
| Carbopol 980 commercialisé par la société GOODRICH | 0,6 g |
| Acide oléique | 2,6 g |
| Aculyn 22 vendu par ROHM & HAAS | 1,4 g M.A. |
| Monoisopropanolamide d'acides de coprah | 3 g |
| Polymère cationique * | 4 g M.A. |
| Hexylène glycol | 6 g |
| Métabisulfite de sodium | 0,71 g |
| EDTA | 0,2 g |
| Terbutyl hydroquinone | 0,3 g |
| 2,3-diamino-6,7-dihydro-1H,5H-pyrazolo[1,2-a]pyrazol-1-one, HCl | 2,27 g |
| 5-amino-6-chloro-o-crésol | 1,58 g |
| Monoéthanolamine | 1 g |
| Ammoniaque à 20 % de NH₃ | 11 g |
| Parfum | q.s. |
| Eau déminéralisée | q.s.p 100 g |
| * Polymère cationique constitué par l'enchaînement de motifs : | |

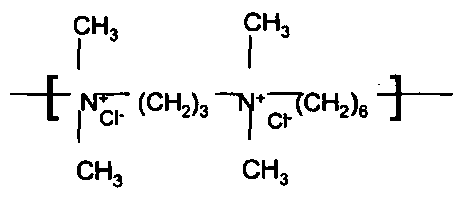

### Mode d'application

Au moment de l'emploi, la composition 1 est mélangée avec 1,5 fois son volume de solution de peroxyde d'hydrogène à 25 volumes dont le pH est égal à 3. On obtient un pH final de 9,8.

Le mélange obtenu est appliqué sur des mèches de cheveux gris à 90 % de blancs naturels à raison de 30 g de mélange pour 3 g de cheveux. Après 30 minutes de pose à température ambiante, les mèches sont rincées, lavées avec un shampooing standard, rincées à nouveau puis séchées.

La coloration capillaire est évaluée de manière visuelle.

| | **Hauteur de ton** | **Reflet** |
|---|---|---|
| Composition 1 | Blond foncé | Cuivré intense |

### Exemple 2 :

La composition 2 suivante a été réalisée :

| | |
|---|---|
| Mélange d'alcools linéaires en C₁₈ à C₂₄ [C₁₈/C₂₀/C₂₂/C₂₄: 7/58/30/6, teneur en alcools > 95 %] | 3 g |
| Mélange d'alcools linéaires en C₁₈ à C₂₄ [C₁₈/C₂₀/C₂₂/C₂₄ : 7/58/30/6, teneur en alcools > 95 %] oxyéthylénés (30 OE) | 1 g |
| Alcool stéarylique oxyéthyléné (2 OE) | 4,5 g |
| Acide stéarylique oxyéthyléné (21 OE) | 1,75 g |
| Acide polyacrylique réticulé : | |
| Carbopol 980 commercialisé par la société GOODRICH | 0,6 g |
| Acide oléique | 2,6 g |
| Aculyn 22 vendu par ROHM & HAAS | 1,4 g M.A. |
| Monoisopropanolamide d'acides de coprah | 3 g |
| Polymère cationique * | 4 g M.A. |
| Néopentyl glycol | 6 g |
| Métabisulfite de sodium | 0,71 g |
| EDTA | 0,2 g |
| Terbutyl hydroquinone | 0,3 g |
| 2,3-diamino-6,7-dihydro-1H,5H-pyrazolo[1,2-a]pyrazol-1-one, HCl | 1,82 g |
| Dichlorhydrate de 2,4-diaminophénoxyéthanol | 1,93 g |
| Monoéthanolamine | 1 g |
| Ammoniaque à 20 % de NH₃ | 11 g |
| Parfum | q.s. |
| Eau déminéralisée | q.s.p 100 g |

| | |
|---|---|
| * Idem exemple 1 | |

### Mode d'application

Au moment de l'emploi, la composition 2 est mélangée avec 1,5 fois son volume de solution de peroxyde d'hydrogène à 25 volumes dont le pH est égal à 3. On obtient un pH final de 9,8.

Le mélange obtenu est appliqué sur des mèches de cheveux gris à 90 % de blancs naturels à raison de 30 g de mélange pour 3 g de cheveux. Après 30 minutes de pose à température ambiante, les mèches sont rincées, lavées avec un shampooing standard, rincées à nouveau puis séchées.

La coloration capillaire est évaluée de manière visuelle.

| | **Hauteur de ton** | **Reflet** |
|---|---|---|
| Composition 2 | Châtain clair | Rouge acajou cuivré |

### Exemple 3 :

La composition 3 suivante a été réalisée :

| | |
|---|---|
| Mélange d'alcools linéaires en C₁₈ à C₂₄ [C₁₈/C₂₀/C₂₂/C₂₄: 7/58/30/6, teneur en alcools > 95 %] | 3 g |
| Mélange d'alcools linéaires en C₁₈ à C₂₄ [C₁₈/C₂₀/C₂₂/C₂₄ : 7/58/30/6, teneur en alcools > 95 %] oxyéthylénés (30 OE) | 1 g |
| Alcool stéarylique oxyéthyléné (2 OE) | 4,5 g |
| Acide stéarylique oxyéthyléné (21 OE) | 1,75 g |
| Acide polyacrylique réticulé : | |
| Carbopol 980 commercialisé par la société GOODRICH | 0,6 g |
| Acide oléique | 2,6 g |
| Aculyn 22 vendu par ROHM & HAAS | 1,4 g M.A. |
| Monoisopropanolamide d'acides de coprah | 3 g |
| Polymère cationique * | 4 g M.A. |
| Hexylène glycol | 6 g |
| Métabisulfite de sodium | 0,71 g |
| EDTA | 0,2 g |
| Terbutyl hydroquinone | 0,3 g |
| 2,3-diamino-6,7-dihydro-1H,5H-pyrazolo[1 ,2-a]pyrazol-1-one, HCl | 1.39 g |
| 5-amino-6-chloro-o-crésol | 0.63 g |
| Monoéthanolamine | 1 g |
| Acide citrique | 0.15 g |
| Parfum | q.s. |
| Eau déminéralisée | q.s.p 100 g |

| | |
|---|---|
| * Polymère cationique constitué par l'enchaînement de motifs : | |

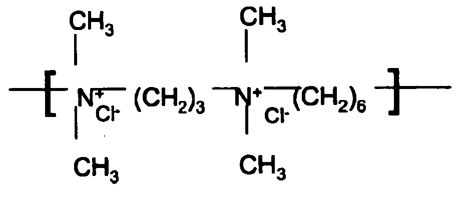

### Mode d'application

Au moment de l'emploi, la composition 3 est mélangée avec 1,5 fois son volume de solution de peroxyde d'hydrogène à 25 volumes dont le pH est égal à 3. On obtient un pH final de 6,85.

Le mélange obtenu est appliqué sur des mèches de cheveux gris à 90 % de blancs naturels à raison de 30 g de mélange pour 3 g de cheveux. Après 30 minutes de pose à température ambiante, les mèches sont rincées, lavées avec un shampooing standard, rincées à nouveau puis séchées.

La coloration capillaire est évaluée de manière visuelle.

| | **Hauteur de ton** | **Reflet** |
|---|---|---|
| Composition 3 | Blond clair | Cuivré doré |

## Revendications

1. Composition de coloration des fibres kératiniques comprenant, dans un milieu approprié :
• au moins une base d'oxydation choisie parmi un dérivé de la diamino-N,N-dihydro-pyrazolone de formule (I) ou l'un de ses sels d'addition : 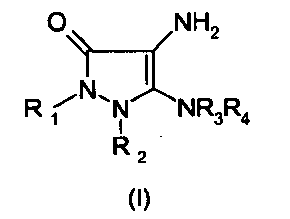
dans laquelle :
R₁, R₂, R₃ et R₄, identiques ou différents, représentent :
- un radical alkyle en C₁-C₁₀ linéaire ou ramifié éventuellement substitué par un ou plusieurs radicaux choisis dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un ou plusieurs groupes (C₁-C₄)alkyle, hydroxy, alcoxy en C₁-C₂, amino, (di)alkyl(C₁-C₂)amino ;
- un radical aryle éventuellement substitué par un ou plusieurs radicaux (C₁-C₄)alkyle, hydroxy, alcoxy en C₁-C₂, amino, (di)alkyl(C₁-C₂)amino ;
- un radical hétéroaryle à 5 ou 6 chaînons, éventuellement substitué par un ou plusieurs radicaux choisis parmi un radical (C₁-C₄)alkyle, (C₁-C₂)alcoxy ;
R₃ et R₄ peuvent représenter également un atome d'hydrogène ;
R₅, R₆ et R₇, identiques ou différents, représentent :
- un atome d'hydrogène ;
- un radical alkyle linéaire ou ramifié en C₁-C₄ éventuellement substitué par un ou plusieurs radicaux choisis parmi un radical hydroxy, alcoxy en C₁-C₂, carboxamido CONR₈R₉, sulfonyle SO₂R₈, aryle éventuellement substitué par un radical (C₁-C₄)alkyle, hydroxy, alcoxy en C₁-C₂, amino, (di)alkyl(C₁-C₂)amino; aryle éventuellement substitué par un radical (C₁-C₄)alkyle, hydroxy, alcoxy en C₁-C₂, amino, (di)alkyl(C₁-C₂)amino ;
R₆ et R₇, identiques ou différents, peuvent représenter également un radical carboxamido CONR₈R₉; un radical sulfonyle SO₂R₈;
R₈ et R₉, identiques ou différents, représentent un atome d'hydrogène ; un radical alkyle en C₁-C₄ linéaire ou ramifié éventuellement substitué par un ou plusieurs radicaux hydroxy, alcoxy en C₁-C₂ ;
R₁ et R₂ d'une part, et R₃ et R₄ d'autre part, peuvent former avec le ou les atomes d'azote auxquels ils sont rattachés un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyle en C₁-C₄ éventuellement substitués par un ou plusieurs radicaux hydroxy, amino, (di)-alkylamino, alcoxy, carboxy, sulfonyle ;
R₃ et R₄ peuvent également former ensemble avec l'atome d'azote auquel ils sont rattachés un hétérocycle à 5 ou 7 chaînons dont les atomes de carbone peuvent être remplacés par un atome d'oxygène ou d'azote éventuellement substitué ;
• au moins un coupleur ; et
• au moins un polyol en C₄-C₃₀ comportant une chaîne hydrocarbonée, linéaire, ramifiée ou cyclique, saturée ou insaturée, portant au moins deux fonctions hydroxyle, ladite chaîne et ses ramifications étant éventuellement interrompues par 1 à 6 atomes d'oxygène et ladite chaîne et ses ramifications étant éventuellement substitués par un ou plusieurs substituants autres que les groupements hydroxyle, substituants choisis parmi les groupements carboxy, amino, halogène, aryle en C₆-C₃₀.

2. Composition selon la revendication 1 dans laquelle R₁ et R₂ sont choisis parmi un radical alkyle en C₁-C₆ éventuellement substitué par un radical hydroxy, (C₁-C₂)alcoxy, amino, (di)alkyl(C₁-C₂)amino; un radical phényle, méthoxyphényle, éthoxyphényle, benzyle.

3. Composition selon la revendication 2 dans laquelle R₁ et R₂ sont choisis parmi un radical méthyle, éthyle, 2-hydroxyéthyle, 3-hydroxypropyle, 2-hydroxypropyle, phényle.

4. Composition selon la revendication 1 dans laquelle R₁ et R₂ forment ensemble avec les atomes d'azote auxquels ils sont rattachés un cycle à 5 ou 6 chaînons, saturé ou insaturé, éventuellement substitué.

5. Composition selon l'une quelconque des revendications 1 et 4 dans laquelle R₁ et R₂ forment ensemble avec les atomes d'azote auxquels ils sont rattachés un cycle pyrazolidine, pyridazolidine, éventuellement substitué par un ou plusieurs radicaux alkyle en C₁-C₄, hydroxy, (C₁-C₂)alcoxy, carboxy, carboxamido, amino, (di)alkyl(C₁-C₂)amino.

6. Composition selon l'une quelconque des revendications 1 et 4 à 5 dans laquelle R₁ et R₂ forment ensemble avec les atomes d'azote auxquels ils sont rattachés un cycle pyrazolidine, pyridazolidine.

7. Composition selon l'une quelconque des revendications précédentes
dans laquelle R₃ et R₄ sont choisis parmi un atome d'hydrogène ; un radical alkyle en C₁-C₆, linéaire ou ramifié, éventuellement substitué par un ou plusieurs radicaux hydroxy, (C₁-C₂)alcoxy, amino, (di)alkyl(C₁-C₂)amino; un radical phényle éventuellement substitué par un ou plusieurs radicaux hydroxy, amino, (C₁-C₂)alcoxy.

8. Composition selon l'une quelconque des revendications 1 à 7 dans laquelle R₃ et R₄ sont choisis parmi un atome d'hydrogène, un radical méthyle, éthyle, isopropyle, 2-hydroxyéthyle, 3-hydroxypropyle, 2-hydroxypropyle, 2-carboxyéthyle.

9. Composition selon la revendication 8 dans laquelle R₃ et R₄ représentent un atome d'hydrogène.

10. Composition selon l'une quelconque des revendications 1 à 6 dans laquelle R₃ et R₄ forment ensemble avec l'atome d'azote auquel ils sont rattachés un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl(C₁-C₂)amino, carboxy, carboxamido, alkyle en C₁-C₄ éventuellement substitué par un ou plusieurs radicaux hydroxy, amino, (di)alkylamino en C₁-C₂.

11. Composition selon l'une quelconque des revendications 1 à 6 et 10
dans laquelle R₃ et R₄ forment ensemble avec l'atome d'azote auquel ils sont rattachés un cycle à 5 ou 7 chaînons choisi parmi la pyrrolidine, le 2,5-diméthylpyrrolidine, l'acide pymolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthylpyrrolidine, la 3-hydroxypyrrolidine, la 3,4-dihydroxypyrrolidine, la 3-aminopyrrolidine, la 3-méthylaminopyrrolidine, la 3-diméthylamino-pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

12. Composition selon l'une quelconque des revendications 1 à 6 et 10 à 11 dans laquelle R₃ et R₄ forment ensemble avec l'atome d'azote auquel ils sont rattachés un cycle à 5 ou 7 chaînons choisi parmi la pyrrolidine, la 3-hydroxypyrrolidine, la 3-aminopymolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthylhomopipérazine, la N β-hydroxyéthylhomopipérazine.

13. Composition selon l'une quelconque des revendications 1 à 6 et 10 à 12 dans laquelle R₃ et R₄ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

14. Composition selon la revendication 1 à 13 dans laquelle le composé de formule (I) ou un de ses sels d'addition est choisi parmi :
2,3-Diamino-6,7-dihydro-1H,5H-pyrazolo[1,2-a]pyrazol-1-one ;
2-Amino-3-éthylamino-6,7-dihydro-1H,5H-pyrazolo[1,2-a]pyrazol-1-one ;
2-Amino-3-isopropylamino-6,7-dihydro-1H,5H-pyrazolo[1,2-a]pyrazol-1-one ;
2-Amino-3-(pyrrolidin-1-yl)-6,7-dihydro-1H,5H-pyrazolo[1,2-a]pyrazol-1-one ;
4,5-diamino-1,2-diméthyl-1,2-dihydro-pyrazol-3-one ;
4,5-diamino-1,2-diéthyl-1,2-dihydro-pyrazol-3-one ;
4,5-diamino-1,2-di-(2-hydroxyéthyl)-1,2-dihydro-pyrazol-3-one ;
2-amino-3-(2-hydroxyéthyl)amino-6,7-dihydro-1H,5H-pyrazolo[1,2-a]pyrazol-1-one ;
2-amino-3-diméthylamino-6,7-dihydro-1H,5H-pyrazolo[1,2-a]pyrazol-1-one;
2,3-diamino-5,6,7,8-tétrahydro-1H,6H-pyridazino[1,2-a]pyrazol-1-one
4-Amino-1.2-diethyl-5-pyrrolidin-1-yl-1,2-dihydro-pyrazol-3-one ;
4-Amino-5-(3-dimethylamino-pyrrolidin-1-yl)-1,2-diethyl-1,2-dihydro-pyrazol-3-one ;
2,3-diamino-6-hydroxy-6,7-dihydro-1H,5H-pyrazolo[1,2-a]pyrazol-1-one.

15. Composition selon l'une quelconque des revendications précédentes
dans laquelle le ou les coupleurs sont choisis parmi les méta-phénylènediamines, les méta-aminophénols, les méta-diphénols, les coupleurs naphtaléniques, les coupleurs hétérocycliques et leurs sels d'addition.

16. Composition selon la revendication 15 dans laquelle le ou les coupleurs sont choisis parmi le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

17. Composition selon la revendication 15 ou 16 dans laquelle la quantité de chacun des coupleurs est comprise entre 0,001 et 10 % en poids du poids total de la composition tinctoriale.

18. Composition selon l'une quelconque des revendications précédentes
dans laquelle le ou les polyols sont en C₄-C₁₅.

19. Composition selon l'une quelconque des revendications précédentes
dans laquelle la chaîne hydrocarboné du polyol et / ou ses ramifications sont interrompues par 1 à 6 atomes d'oxygène.

20. Composition selon la revendication 19 dans laquelle le ou les polyols sont choisis parmi le polyéthylène glycol à 4, 6 ou 7 motifs d'éthylène ou le dipropylène glycol.

21. Composition selon l'une quelconque des revendications 1 à 18 dans laquelle la chaîne hydrocarboné du polyol et ses ramifications ne sont pas interrompues par des atomes d'oxygène.

22. Composition selon la revendication 21 dans laquelle le ou les polyols sont choisis parmi l'hexylèneglycol ; le néopentylglycol ; l'isoprèneglycol.

23. Composition selon l'une quelconque des revendications précédentes
dans laquelle le ou les polyols sont présents dans des proportions comprises entre 0,1 et 40 % en poids du poids total de la composition tinctoriale.

24. Composition selon l'une quelconque des revendications précédentes comprenant une base d'oxydation additionnelle choisie parmi les paraphénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis dans l'une quelconque des revendications 1 à 14 et leurs sels d'addition, ainsi que leurs mélanges.

25. Composition selon l'une quelconque des revendications précédentes
dans laquelle la quantité de chacune des bases d'oxydation est comprise entre 0,001 et 10 % en poids du poids total de la composition tinctoriale.

26. Composition selon l'une quelconque des revendications précédentes comprenant de plus un agent oxydant.

27. Procédé de teinture des fibres kératiniques, **caractérisé en ce qu'**une composition telle que définie dans l'une quelconque des revendications 1 à 25 est appliquée sur les fibres kératiniques en présence d'un agent oxydant pendant un temps suffisant pour développer la coloration désirée.

28. Procédé selon la revendication 27 dans lequel l'agent oxydant est choisi parmi le peroxyde d'hydrogène, le peroxyde d'urée, les bromates de métaux alcalins, les persels, les peracides et les enzymes oxydases.

29. Dispositif à plusieurs compartiments dans lequel un premier compartiment contient une composition tinctoriale telle que définie dans l'une quelconque des revendications 1 à 25 et un deuxième compartiment contient un agent oxydant.

30. Utilisation pour la teinture d'oxydation des fibres kératiniques d'une composition telle que définie dans l'une quelconque des revendications 1 à 26.

## Claims

1. Composition for dyeing keratin fibres, comprising, in a suitable medium:
• at least one oxidation base chosen from a diamino-N,N-dihydropyrazolone derivative of formula (I), or an addition salt thereof: 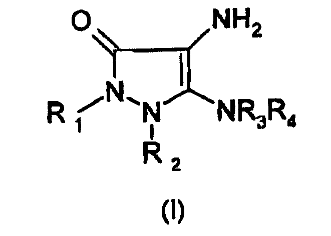
in which:
R₁, R₂, R₃ and R₄, which may be identical or different, represent:
- a linear or branched C₁-C₁₀ alkyl radical optionally substituted with one or more radicals chosen from the group consisting of a radical OR₅, a radical NR₆R₇, a carboxyl radical, a sulfonic radical, a carboxamido radical CONR₆R₇, a sulfonamido radical SO₂NR₆R₇, a heteroaryl or an aryl optionally substituted with one or more (C₁-C₄)alkyl, hydroxyl, C₁-C₂ alkoxy, amino or (di) (C₁-C₂)alkylamino groups;
- an aryl radical optionally substituted with one or more (C₁-C₄)alkyl, hydroxyl, C₁-C₂ alkoxy, amino or (di)(C₁-C₂)alkylamino radicals;
- a 5- or 6-membered heteroaryl radical, optionally substituted with one or more radicals chosen from a (C₁-C₄)alkyl or (C₁-C₂)alkoxy radical;
R₃ and R₄ may also represent a hydrogen atom;
R₅, R₆ and R₇, which may be identical or different, represent:
- a hydrogen atom;
- a linear or branched C₁-C₄ alkyl radical optionally substituted with one or more radicals chosen from a hydroxyl, C₁-C₂ alkoxy, carboxamido CONR₈R₉, sulfonyl SO₂R₈ or aryl radical optionally substituted with a (C₁-C₄)alkyl, hydroxyl, C₁-C₂ alkoxy, amino or (di) (C₁-C₂) alkylamino radical; aryl optionally substituted with a (C₁-C₄)alkyl, hydroxyl, C₁-C₂ alkoxy, amino or (di)(C₁-C₂)alkylamino radical;
R₆ and R₇, which may be identical or different, may also represent a carboxamido radical CONR₈R₉; a sulfonyl radical SO₂R₈;
R₈ and R₉, which may be identical or different, represent a hydrogen atom; a linear or branched C₁-C₄ alkyl radical optionally substituted with one or more hydroxyl or C₁-C₂ alkoxy radicals;
R₁ and R₂, on the one hand, and R₃ and R₄, on the other hand, may form, with the nitrogen atom(s) to which they are attached, a saturated or unsaturated 5- to 7-membered heterocycle optionally substituted with one or more radicals chosen from the group consisting of halogen atoms, amino, (di)(C₁-C₄)alkylamino, hydroxyl, carboxyl, carboxamido or (C₁-C₂)alkoxy radicals, or C₁-C₄ alkyl radicals optionally substituted with one or more hydroxyl, amino, (di)alkylamino, alkoxy, carboxyl or sulfonyl radicals;
R₃ and R₄ may also form, together with the nitrogen atom to which they are attached, a 5- or 7-membered heterocycle, the carbon atoms of which may be replaced with an optionally substituted oxygen or nitrogen atom;
• at least one coupler; and
• at least one C₄-C₃₀ polyol comprising a saturated or unsaturated, linear, branched or cyclic hydrocarbon-based chain, bearing at least two hydroxyl functions, the said chain and its branches being optionally interrupted with 1 to 6 oxygen atoms and the said chain and its branches being optionally substituted with one or more substituents other than hydroxyl groups, these substituents being chosen from carboxyl, amino, halogen and C₆-C₃₀ aryl groups.

2. Composition according to Claim 1, in which R₁ and R₂ are chosen from a C₁-C₆ alkyl radical optionally substituted with a hydroxyl, (C₁-C₂)alkoxy, amino or (di)(C₁-C₂)alkylamino radical; a phenyl, methoxyphenyl, ethoxyphenyl or benzyl radical.

3. Composition according to Claim 2, in which R₁ and R₂ are chosen from methyl, ethyl, 2-hydroxyethyl, 3-hydroxypropyl, 2-hydroxypropyl and phenyl radicals.

4. Composition according to Claim 1, in which R₁ and R₂ form, together with the nitrogen atoms to which they are attached, a saturated or unsaturated, optionally substituted 5- or 6-membered ring.

5. Composition according to either of Claims 1 and 4, in which R₁ and R₂ form, together with the nitrogen atoms to which they are attached, a pyrazolidine or pyridazolidine ring, optionally substituted with one or more C₁-C₄ alkyl, hydroxyl, (C₁-C₂)alkoxy, carboxyl, carboxamido, amino or (di) (C₁-C₂)alkylamino radicals.

6. Composition according to any one of Claims 1, 4 and 5, in which R₁ and R₂ form, together with the nitrogen atoms to which they are attached, a pyrazolidine or pyridazolidine ring.

7. Composition according to any one of the preceding claims, in which R₃ and R₄ are chosen from a hydrogen atom; a linear or branched C₁-C₆ alkyl radical optionally substituted with one or more hydroxyl, (C₁-C₂)alkoxy, amino or (di)(C₁-C₂)alkylamino radicals; a phenyl radical optionally substituted with one or more hydroxyl, amino or (C₁-C₂)alkoxy radicals.

8. Composition according to any one of Claims 1 to 7, in which R₃ and R₄ are chosen from a hydrogen atom and a methyl, ethyl, isopropyl, 2-hydroxyethyl, 3-hydroxypropyl, 2-hydroxypropyl or 2-carboxyethyl radical.

9. Composition according to Claim 8, in which R₃ and R₄ represent a hydrogen atom.

10. Composition according to any one of Claims 1 to 6, in which R₃ and R₄ form, together with the nitrogen atom to which they are attached, a 5- or 7-membered ring chosen from pyrrolidine, piperidine, homopiperidine, piperazine and homopiperazine heterocycles; the said rings possibly being substituted with one or more hydroxyl, amino, (di)(C₁-C₂)alkylamino, carboxyl, carboxamido or C₁-C₄ alkyl radicals optionally substituted with one or more hydroxyl, amino or C₁-C₂ (di)alkylamino radicals.

11. Composition according to any one of Claims 1 to 6 and 10, in which R₃ and R₄ form, together with the nitrogen atom to which they are attached, a 5- or 7-membered ring chosen from pyrrolidine, 2,5-dimethyl-pyrrolidine, pyrrolidine-2-carboxylic acid, 3-hydroxypyrrolidine-2-carboxylic acid, 4-hydroxypyrrolidine-2-carboxylic acid, 2,4-dicarboxypyrrolidine, 3-hydroxy-2-hydroxymethylpyrrolidine, 2-carboxamidopyrrolidine, 3-hydroxy-2-carboxamidopyrrolidine, 2-(diethylcarboxamido)pyrrolidine, 2-hydroxymethylpyrrolidine, 3,4-dihydroxy-2-hydroxymethylpyrrolidine, 3-hydroxypyrrolidine, 3,4-dihydroxypyrrolidine, 3-aminopyrrolidine, 3-methylaminopyrrolidine, 3-dimethylaminopyrrolidine, 4-amino-3-hydroxypyrrolidine, 3-hydroxy-4-(2-hydroxyethyl)-aminopyrrolidine, piperidine, 2,6-dimethylpiperidine, 2-carboxypiperidine, 2-carboxamidopiperidine, 2-hydroxymethylpiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, homopiperazine, N-methylhomopiperazine and N-(2-hydroxyethyl)homopiperazine.

12. Composition according to any one of Claims 1 to 6, 10 and 11, in which R₃ and R₄ form, together with the nitrogen atom to which they are attached, a 5- or 7-membered ring chosen from pyrrolidine, 3-hydroxypyrrolidine, 3-aminopyrrolidine, 3-dimethylaminopyrrolidine, pyrrolidine-2-carboxylic acid, 3-hydroxypyrrolidine-2-carboxylic acid, piperidine, hydroxypiperidine, homopiperidine, diazepane, N-methylhomopiperazine and N-β-hydroxyethylhomopiperazine.

13. Composition according to any one of Claims 1 to 6 and 10 to 12, in which R₃ and R₄ form, together with the nitrogen atom to which they are attached, a 5-membered ring such as pyrrolidine, 3-hydroxypyrrolidine, 3-aminopyrrolidine or 3-dimethylaminopyrrolidine.

14. Composition according to Claims 1 to 13, in which the compound of formula (I), or an addition salt thereof, is chosen from:
2,3-diamino-6,7-dihydro-1H,5H-pyrazolo[1,2-a]pyrazol-1-one;
2-amino-3-ethylamino-6,7-dihydro-1H,5H-pyrazolo-[1,2-a]pyrazol-1-one;
2-amino-3-isopropylamino-6,7-dihydro-1H,5H-pyrazolo-[1,2-a]pyrazol-1-one;
2-amino-3-(pyrrolidin-1-yl)-6,7-dihydro-1H,5H-pyrazolo-[1,2-a]pyrazol-1-one;
4,5-diamino-1,2-dimethyl-1,2-dihydropyrazol-3-one;
4,5-diamino-1,2-diethyl-1,2-dihydropyrazol-3-one;
4,5-diamino-1,2-di-(2-hydroxyethyl)-1,2-dihydropyrazol-3-one;
2-amino-3-(2-hydroxyethyl)amino-6,7-dihydro-1H,5H-pyrazolo[1,2-a]pyrazol-1-one;
2-amino-3-dimethylamino-6,7-dihydro-1H,5H-pyrazolo-[1,2-a]pyrazol-1-one;
2,3-diamino-5,6,7,8-tetrahydro-1H,6H-pyridazino-[1,2-a]pyrazol-1-one;
4-amino-1,2-diethyl-5-pyrrolidin-1-yl-1,2-dihydropyrazol-3-one;
4-amino-5-(3-dimethylaminopyrrolidin-1-yl)-1,2-diethyl-1,2-dihydropyrazol-3-one;
2,3-diamino-6-hydroxy-6,7-dihydro-1H,5H-pyrazolo-[1,2-a]pyrazol-1-one.

15. Composition according to any one of the preceding claims, in which the coupler(s) is(are) chosen from meta-phenylenediamines, meta-aminophenols, metadiphenols, naphthalene-based couplers and heterocyclic couplers, and the addition salts thereof.

16. Composition according to Claim 15, in which the coupler(s) is(are) chosen from 2-methyl-5-aminophenol, 5-N-(β-hydroxyethyl)amino-2-methylphenol, 6-chloro-2-methyl-5-aminophenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)-benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene and 2,6-bis(β-hydroxyethylamino)toluene, and the addition salts thereof with an acid.

17. Composition according to Claim 15 or 16, in which the amount of each of the couplers is between 0.001% and 10% by weight relative to the total weight of the dye composition.

18. Composition according to any one of the preceding claims, in which the polyol(s) is (are) C₄-C₁₅.

19. Composition according to any one of the preceding claims, in which the hydrocarbon-based chain of the polyol and/or its branches is interrupted with 1 to 6 oxygen atoms.

20. Composition according to Claim 19, in which the polyol(s) is (are) chosen from polyethylene glycol containing 4, 6 or 7 ethylene units or dipropylene glycol.

21. Composition according to any one of Claims 1 to 18, in which the hydrocarbon-based chain of the polyol and its branches is not interrupted with oxygen atoms.

22. Composition according to Claim 21, in which the polyol(s) is (are) chosen from hexylene glycol; neopentyl glycol; isoprene glycol.

23. Composition according to any one of the preceding claims, in which the polyol(s) is (are) present in proportions of between 0.1% and 40% by weight relative to the total weight of the dye composition.

24. Composition according to any one of the preceding claims, comprising an additional oxidation base chosen from para-phenylenediamines, bis(phenyl)alkylenediamines, para-aminophenols, bis-para-aminophenols, ortho-aminophenols, ortho-phenylenediamines, heterocyclic bases other than the derivatives of formula (I) as defined in any one of Claims 1 to 14, and the addition salts thereof, and also mixtures thereof.

25. Composition according to any one of the preceding claims, in which the amount of each of the oxidation bases is between 0.001% and 10% by weight relative to the total weight of the dye composition.

26. Composition according to any one of the preceding claims, also comprising an oxidizing agent.

27. Process for dyeing keratin fibres, **characterized in that** a composition as defined in any one of Claims 1 to 25 is applied to the keratin fibres in the presence of an oxidizing agent for a time that is sufficient to develop the desired coloration.

28. Process according to Claim 27, in which the oxidizing agent is chosen from hydrogen peroxide, urea peroxide, alkali metal bromates, persalts, peracids and oxidase enzymes.

29. Multi-compartment device, in which a first compartment contains a dye composition as defined in any one of Claims 1 to 25 and a second compartment contains an oxidizing agent.

30. Use for the oxidation dyeing of keratin fibres of a composition as defined in any one of Claims 1 to 26.

## Patentansprüche

1. Zusammensetzung zum Färben von Keratinfasern, die in einem geeigneten Medium enthält:
○ mindestens eine Oxidationsbase, die unter den Diamino-N,N-dihydro-pyrazolonderivaten der Formel (I) oder einem ihrer Additionssalze ausgewählt ist: 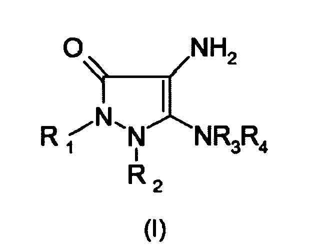 in der Formel:
R₁, R₂, R₃ und R₄, die gleich oder verschieden sind, bedeuten:
- eine geradkettige oder verzweigte C₁₋₁₀-Alkylgruppe, die gegebenenfalls mit einer oder mehreren Gruppen substituiert ist, die unter einer Gruppe OR₅, einer Gruppe NR₆R₇, einer Carboxygruppe, einer Sulfonsäuregruppe, einer Carboxamidogruppe CONR₆R₇, einer Sulfonamidogruppe SO₂NR₆R₇, einer Heteroarylgruppe und einer Arylgruppe ausgewählt sind, die gegebenenfalls mit einer oder mehreren Gruppen (C₁₋₄)-Alkyl, Hydroxy, (C₁₋₂)Alkoxy, Amino, (Di)alkyl(C₁₋₂)amino substituiert ist;
- eine Arylgruppe, die gegebenenfalls mit einer oder mehreren Gruppen (C₁₋₄)Alkyl, Hydroxy, (C₁₋₂)Alkoxy, Amino, (Di)alkyl(C₁₋₂)amino substituiert ist;
- eine 5- oder 6-gliedrige Heteroarylgruppe, die gegebenenfalls mit einer oder mehreren Gruppen substituiert ist, die unter (C₁₋₄)Alkyl und (C₁₋₂)Alkoxy ausgewählt sind;
R₃ und R₄ können auch ein Wasserstoffatom bedeuten;
R₅, R₆ und R₇, die gleich oder verschieden sind, bedeuten:
- ein Wasserstoffatom;
- eine geradkettige oder verzweigte C₁₋₄-Alkylgruppe, die gegebenenfalls mit einer oder mehreren Gruppen substituiert ist, die unter Hydroxy, (C₁₋₂)Alkoxy, Carboxamido CONR₈R₉, Sulfonyl SO₂R₈ und einer Arylgruppe ausgewählt sind, die gegebenenfalls mit (C₁₋₄)Alkyl, Hydroxy, (C₁₋₂)Alkoxy, Amino, (Di)alkyl(C₁₋₂)amino substituiert ist; eine Arylgruppe, die gegebenenfalls mit (C₁₋₄)Alkyl, Hydroxy, (C₁₋₂)Alkoxy, Amino, (Di)alkyl(C₁₋₂)amino substituiert ist;
R₆ und R₇, die gleich oder verschieden sind, können auch eine Carboxamidogruppe CONR₈R₉; eine Sulfonylgruppe SO₂R₈ bedeuten;
R₈ und R₉, die gleich oder verschieden sind, bedeuten ein Wasserstoffatom; eine geradkettige oder verzweigte C₁₋₄-Alkylgruppe, die gegebenenfalls mit einer oder mehreren Gruppen Hydroxy, (C₁₋₂)Alkoxy substituiert ist;
R₁ und R₂ einerseits und R₃ und R₄ andererseits können mit dem oder den Stickstoffatom(en), an die sie gebunden sind, einen gesättigten oder ungesättigten, 5- bis 7-gliedrigen Heterocyclus bilden, der gegebenenfalls mit einer oder mehreren Gruppen substituiert ist, die unter den Halogenatomen, den Gruppen Amino, (Di)alkyl(C₁₋₄)amino, Hydroxy, Carboxy, Carboxamido, (C₁₋₂)-Alkoxy und den C₁₋₄-Alkylgruppen ausgewählt sind, die gegebenenfalls mit einer oder mehreren Gruppen Hydroxy, Amino, (Di)alkylamino, Alkoxy, Carboxy, Sulfonyl substituiert sind;
R₃ und R₄ können auch gemeinsam mit dem Stickstoffatom, an das sie gebunden sind, einen 5- oder 7-gliedrigen Heterocyclus bilden, bei dem die Kohlenstoffatome durch ein Sauerstoffatom oder ein Stickstoffatom ersetzt sein können, das gegebenenfalls substituiert ist;
○ mindestens einen Kuppler; und
○ mindestens ein C₄₋₃₀-Polyol, das eine lineare, verzweigte oder cyclische, gesättigte oder ungesättigte Kohlenwasserstoffkette aufweist, die mindestens zwei Hydroxyfunktionen trägt, wobei die Kette und ihre Verzweigungen gegebenenfalls durch 1 bis 6 Sauerstoffatome unterbrochen sein können und wobei die Kette und ihre Verzweigungen gegebenenfalls mit einem oder mehreren, von Hydroxygruppen verschiedenen Substituenten substituiert sein können, die unter den Gruppen Carboxy, Amino, Halogen, (C₆₋₃₀)Aryl ausgewählt sind.

2. Zusammensetzung nach Anspruch 1, bei der die Gruppen R₁ und R₂ unter einer Alkyl(C₁-₆)gruppe, die gegebenenfalls mit Hydroxy, (C₁₋₂)Alkoxy, Amino, (Di)alkyl(C₁₋₂)amino substituiert ist; einer Phenylgruppe, einer Methoxyphenylgruppe, einer Ethoxyphenygruppe, einer Benzylgruppe ausgewählt sind.

3. Zusammensetzung nach Anspruch 2, bei der die Gruppen R₁ und R₂ unter Methyl, Ethyl, 2-Hydroxyethyl, 3-Hydroxypropyl, 2-Hydroxypropyl, Phenyl ausgewählt sind.

4. Zusammensetzung nach Anspruch 1, bei der die Gruppen R₁ und R₂ gemeinsam mit den Stickstoffatomen, an die sie gebunden sind, einen 5- oder 6-gliedrigen, gesättigten oder ungesättigten, gegebenenfalls substituierten Ring bilden.

5. Zusammensetzung nach einem der Ansprüche 1 bis 4, bei der die Gruppen R₁ und R₂ gemeinsam mit den Stickstoffatomen, an die sie gebunden sind, einen Pyrazolidinring oder Pyridazolidinring bilden, wobei diese Ringe gegebenenfalls mit einer oder mehreren Gruppen (C₁₋₄)Alkyl, Hydroxy, (C₁₋₂)Alkoxy, Carboxy, Carboxamido, Amino, (Di)alkyl(C₁₋₂)amino substituiert sein können.

6. Zusammensetzung nach einem der Ansprüche 1 und 4 bis 5, bei der die Gruppen R₁ und R₂ gemeinsam mit den Stickstoffatomen, an die sie gebunden sind, einen Pyrazolidinring oder Pyridazolidinring bilden.

7. Zusammensetzung nach einem der vorhergehenden Ansprüche, bei der die Gruppen R₃ und R₄ unter einem Wasserstoffatom; einer geradkettigen oder verzweigten Alkyl(C₁₋₆)gruppe, die gegebenenfalls mit einer oder mehreren Gruppen Hydroxy, (C₁₋₂)Alkoxy, Amino, (Di)alkyl(C₁₋₂)amino substituiert ist; einer Phenylgruppe, die gegebenenfalls mit einer oder mehreren Gruppen Hydroxy, Amino, (C₁₋₂)Alkoxy substituiert ist, ausgewählt sind.

8. Zusammensetzung nach einem der Ansprüche 1 bis 7, bei der die Gruppen R₃ und R₄ unter einem Wasserstoffatom, Methyl, Ethyl, Isopropyl, 2-Hydroxyethyl, 3-Hydroxypropyl, 2-Hydroxypropyl, 2-Carboxyethyl ausgewählt sind.

9. Zusammensetzung nach Anspruch 8, bei der die Gruppen R₃ und R₄ ein Wasserstoffatom bedeuten.

10. Zusammensetzung nach einem der Ansprüche 1 bis 6, bei der die Gruppen R₃ und R₄ gemeinsam mit dem Stickstoffatom, an das sie gebunden sind, einen 5- oder 7-gliedrigen Ring bilden, der unter den Heterocyclen Pyrrolidin, Piperidin, Homopiperidin, Piperazin, Homopiperazin ausgewählt ist; wobei diese Ringe gegebenenfalls mit einer oder mehreren Gruppen Hydroxy, Amino, (Di)alkyl(C₁₋₂)amino, Carboxy, Carboxamido, (C₁₋₄)Alkyl substituiert sein können, wobei die (C₁₋₄)Alkylgruppe gegebenenfalls mit einer oder mehreren Gruppen Hydroxy, Amino, (Di)alkyl(C₁₋₂)amino substituiert sein kann.

11. Zusammensetzung nach einem der Ansprüche 1 bis 6 und 10, bei der die Gruppen R₃ und R₄ gemeinsam mit dem Stickstoffatom, an das sie gebunden sind, einen 5- oder 7- gliedrigen Ring bilden, der unter Pyrrolidin, 2,5-Dimethylpyrrolidin, Pyrrolidin-2-carbonsäure, 3-Hydroxypyrrolidin-2-carbonsäure, 4-Hydroxypyrrolidin-2-carbonsäure, 2,4-Dicarboxypyrrolidin, 3-Hydroxy-2-hydroxymethylpyrrolidin, 2-Carboxamidopyrrolidin, 3-Hydroxy-2-carboxamidopyrrolidin, 2-(Diethylcarboxamido)pyrrolidin, 2-Hydroxymethyl-pyrrolidin, 3,4-Dihydroxy-2-hydroxymethylpyrrolidin, 3-Hydroxypyrrolidin, 3,4-Dihydroxypyrrolidin, 3-Aminopyrrolidin, 3-Methylaminopyrrolidin, 3-Dimethylaminopyrrolidin, 4-Amino-3-hydroxypyrrolidin, 3-Hydroxy-4-(2-hydroxyethyl)aminopyrrolidin, Piperidin, 2,6-Dimethylpiperidin, 2-Carboxypiperidin, 2-Carboxamidopiperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxyhomopiperidin, 2-Carboxamidohomopiperidin, Homopiperazin, N-Methyl-homopiperazin, N-(2-Hydroxyethyl)-homopiperazin ausgewählt ist.

12. Zusammensetzung nach einem der Ansprüche 1 bis 6 und 10 bis 11, bei der die Gruppen R₃ und R₄ gemeinsam mit dem Stickstoffatom, an das sie gebunden sind, einen 5- oder 7- gliedrigen Ring bilden, der unter Pyrrolidin, 3-Hydroxypyrrolidin, 3-Aminopyrrolidin, 3-Dimethylamino-pyrrolidin, Pyrrolidin-2-carbonsäure, 3-Hydroxypyrrolidin-2-carbonsäure, Piperidin, Hydroxypiperidin, Homopiperidin, Diazepan, N-Methylhomopiperazin, N-β-Hydroxyethylhomopiperazin ausgewählt ist.

13. Zusammensetzung nach einem der Ansprüche 1 bis 6 und 10 bis 12, bei der die Gruppen R₃ und R₄ gemeinsam mit dem Stickstoffatom, an das sie gebunden sind, einen 5-gliedrigen Ring bilden, wie Pyrrolidin, 3-Hydroxypyrrolidin, 3-Aminopyrrolidin, 3-Dimethylaminopyrrolidin.

14. Zusammensetzung nach Anspruch 1 bis 13, bei der die Verbindung der Formel (I) oder eines ihrer Additionssalze ausgewählt ist unter:
2,3-Diamino-6,7-dihydro-1H,5H-pyrazolo[1,2-a]pyrazol-1-on;
2-Amino-3-ethylamino-6,7-dihydro-1H,5H-pyrazolo[1,2-a]-pyrazol-1-on;
2-Amino-3-isopropylamino-6,7-dihydro-1H,5H-pyrazolo[1,2-a]-pyrazol-1-on;
2-Amino-3-(pyrrolidin-1-yl)-6,7-dihydro-1H,5H-pyrazolo[1,2-a]-pyrazol-1-on;
4,5-Diamino-1,2-dimethyl-1,2-dihydro-pyrazol-3-on;
4,5-Diamino-1,2-diethyl-1,2-dihydro-pyrazol-3-on;
4,5-Diamino-1,2-di-(2-hydroxyethyl)-1,2-dihydro-pyrazol-3-on;
2-Amino-3-(2-hydroxyethyl)amino-6,7-dihydro-1H,5H-pyrazolo[1,2-a]pyrazol-1-on;
2-Amino-3-dimethylamino-6,7-dihydro-1H,5H-pyrazolo[1,2-a]-pyrazol-1-on;
2,3-Diamino-5,6,7,8-tetrahydro-1H,6H-pyridazino[1,2-a]pyrazol-1-on;
4-Amino-1,2-diethyl-5-pyrrolidin-1-yl-1,2-dihydro-pyrazol-3-on;
4-Amino-5-(3-dimethylamino-pyrrolidin-1-yl)-1,2-diethyl-1,2-dihydro-pyrazol-3-on;
2,3-Diamino-6-hydroxy-6,7-dihydro-1H,5H-pyrazolo[1,2-a]-pyrazol-1-on.

15. Zusammensetzung nach einem der vorhergehenden Ansprüche, wobei der oder die Kuppler unter den meta-Phenylendiaminen, meta-Aminophenolen, meta-Dihydroxybenzolen, Naphthalinkupplern, heterocyclischen Kupplern und deren Additionssalzen ausgewählt sind.

16. Zusammensetzung nach Anspruch 15, wobei der oder die Kuppler unter 2-Methyl-5-amino-phenol, 5-N-(β-Hydroxyethyl)-amino-2-methyl-phenol, 6-Chlor-2-methyl-5-aminophenol, 3-Amino-phenol, 1,3-Dihydroxy-benzol, 1,3-Dihydroxy-2-methylbenzol, 4-Chlor-1,3-dihydroxy-benzol, 2,4-Diamino-1-(β-hydroxyethyloxy)-benzol, 2-Amino-4-(β-hydroxyethylamino)-1-methoxy-benzol, 1,3-Diamino-benzol, 1,3-Bis(2,4-diaminophenoxy)-propan, 3-Ureidoanilin, 3-Ureido-1-dimethylaminobenzol, Sesamol, 1-β-Hydroxyethylamino-3,4-methylendioxybenzol, α-Naphthol, 2-Methyl-1-naphthol, 6-Hydroxy-indol, 4-Hydroxy-indol, 4-Hydroxy-N-methyl-indol, 2-Amino-3-hydroxy-pyridin, 6-Hydroxy-benzomorpholin, 3,5-Diamino-2,6-dimethoxypyridin, 1-N-(β-Hydroxyethyl)amino-3,4-methylendioxy-benzol, 2,6-Bis(β-hydroxyethylamino)-toluol und deren Additionssalzen mit einer Säure ausgewählt sind.

17. Zusammensetzung nach Anspruch 15 oder 16, wobei der Mengenanteil jedes Kupplers im Bereich von 0,001 bis 10 Gew.-% des Gesamtgewichts der Farbmittelzusammensetzung, liegt.

18. Zusammensetzung nach einem der vorhergehenden Ansprüche, wobei das oder die Polyol(e) 4 bis 15 Kohlenstoffatome aufweisen.

19. Zusammensetzung nach einem der vorhergehenden Ansprüche, wobei die hydrophobe Kette des Polyols und/oder seine Verzweigungen durch 1 bis 6 Sauerstoffatome unterbrochen sind.

20. Zusammensetzung nach Anspruch 19, wobei das oder die Polyol(e) unter Polyethylenglycol mit 4, 6 oder 7 Ethyleneinheiten oder Dipropylenglycol ausgewählt ist.

21. Zusammensetzung nach einem der Ansprüche 1 bis 18, wobei die hydrophobe Kette des Polyols und seine Verzweigungen nicht durch Sauerstoffatome unterbrochen sind.

22. Zusammensetzung nach Anspruch 21, wobei das oder die Polyol(e) unter Hexylenglycol; Neopentylglycol; Isoprenglycol ausgewählt sind.

23. Zusammensetzung nach einem der vorhergehenden Ansprüche, bei der das oder die Polyol(e) in einem Mengenanteil im Bereich von 0,1 bis 40 Gew.-% des Gesamtgewichts der Farbmittelzusammensetzung vorliegen.

24. Zusammensetzung nach einem der vorhergehenden Ansprüche, die eine ergänzende Oxidationsbase enthält, die unter den p-Phenylendiaminen, Bisphenylalkylendiaminen, p-Aminophenolen, Bis-p-aminophenolen, o-Aminophenolen, o-Phenylendiaminen, heterocyclischen Basen, die von den Derivaten der Formel (I), wie sie in einem der Ansprüche 1 bis 14 definiert sind, verschieden sind, und deren Additionssalzen und ihren Gemischen ausgewählt ist.

25. Zusammensetzung nach einem der vorhergehenden Ansprüche, bei der der Mengenteil jeder Oxidationsbase im Bereich von 0,001 bis 10 Gew.-% des Gesamtgewichts der Farbmittelzusammensetzung liegt.

26. Zusammensetzung nach einem der vorhergehenden Ansprüche, die ferner ein Oxidationsmittel enthält.

27. Verfahren zum Färben von Keratinfasern, **dadurch gekennzeichnet, dass** eine Zusammensetzung, wie sie in einem der Ansprüche 1 bis 25 definiert ist, in Gegenwart eines Oxidationsmittels während einer Zeitspanne, die ausreichend ist, um die gewünschte Farbe zu bilden, auf die Keratinfasern aufgebracht wird.

28. Verfahren nach Anspruch 27, wobei das Oxidationsmittel unter Wasserstoffperoxid, Harnstoffperoxid, Alkalimetallbromaten, Salzen von Persäuren, Persäuren und Oxidasen ausgewählt ist.

29. Vorrichtung mit mehreren Abteilungen, wobei eine erste Abteilung eine Farbmittelzusammensetzung enthält, wie sie in einem der Ansprüche 1 bis 25 definiert ist, und eine zweite Abteilung ein Oxidationsmittel enthält.

30. Verwendung einer Zusammensetzung nach einem der Ansprüche 1 bis 26 zum oxidativen Färben von Keratinfasern.
